# EUROPEAN PATENT SPECIFICATION

(11) **EP 2 456 767 B1**
(45) Date of publication and mention of the grant of the patent: **12.03.2014**
(21) Application number: 10733008.6
(22) Date of filing: 20.07.2010
(51) Int. Cl.: C07D 403/12, C07D 403/14, A61K 31/415, A61P 3/10

(54) **PYRIDONE GLUCOKINASE ACTIVATORS**
PYRIDONGLUCOKINASE-AKTIVATOREN
ACTIVATEURS DE LA GLUCOKINASE À BASE DE PYRIDINE

(30) Priority: 23.07.2009 US 227874 P
(43) Date of publication of application: 30.05.2012
(73) Proprietor: F.Hoffmann-La Roche AG, 4070 Basel (CH)
(72) Inventor: HAYNES, Nancy-Ellen, Cranford New Jersey 07016 (US); SCOTT, Nathan, Robert, Livingston New Jersey 07039 (US); TILLEY, Jefferson, Wright, North Caldwell New Jersey 07006 (US)
(74) Representative: Salud, Carlos E.
(86) International application number: PCT/EP2010/060452
(87) International publication number: WO 2011/009845

(56) References cited:
- WO-A1-03/095438
- WO-A1-2004/052869
- WO-A1-2009/127546
- WO-A2-2008/012227
- US-B1- 6 528 543

## Description

The invention is directed to compounds of the formula (I): 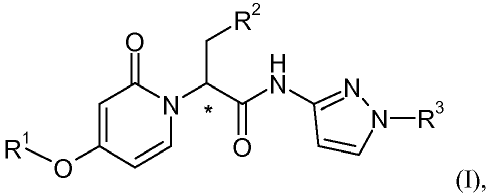 and salts thereof, pharmaceutical compositions containing them and to methods of treating diseases and disorders. The compounds and compositions disclosed herein are glucokinase activators useful for the treatment of metabolic diseases and disorders, preferably diabetes mellitus, more preferably type II diabetes mellitus.

Glucokinase (GK) is one of four hexokinases that are found in mammals (Colowick, S.P., in The Enzymes, Vol. 9 (P. Boyer, ed.) Academic Press, New York, NY, pages 1-48, 1973). The hexokinases catalyze the first step in the metabolism of glucose, i.e., the conversion of glucose to glucose-6-phosphate. Glucokinase has a limited cellular distribution, being found principally in pancreatic β-cells and liver parenchymal cells. In addition, GK is a rate-controlling enzyme for glucose metabolism in these two cell types that are known to play critical roles in whole-body glucose homeostasis (Chipkin, S.R., Kelly, K.L., and Ruderman, N.B. in Joslin's Diabetes (C.R. Khan and G.C. Wier, eds.), Lea and Febiger, Philadelphia, PA, pages 97-115, 1994). The concentration of glucose at which GK demonstrates half-maximal activity is approximately 8 mM. The other three hexokinases are saturated with glucose at much lower concentrations (<1 mM). Therefore, the flux of glucose through the GK pathway rises as the concentration of glucose in the blood increases from fasting (5 mM) to postprandial (≈10-15 mM) levels following a carbohydrate-containing meal (Printz, R.G., Magnuson, M.A., and Granner, D.K. in Ann. Rev. Nutrition Vol. 13 (R.E. Olson, D.M. Bier, and D.B. McCormick, eds.), Annual Review, Inc., Palo Alto, CA, pages 463-496, 1993). These findings contributed over a decade ago to the hypothesis that GK functions as a glucose sensor in β-cells and hepatocytes (Meglasson, M.D. and Matschinsky, F.M. Amer. J. Physiol. 246, E1-E13, 1984). In recent years, studies in transgenic animals have confirmed that GK does indeed play a critical role in whole-body glucose homeostasis. Animals that do not express GK die within days of birth with severe diabetes while animals overexpressing GK have improved glucose tolerance (Grupe, A., Hultgren, B., Ryan, A. et al., Cell 83, 69-78, 1995; Ferrie, T., Riu, E., Bosch, F. et al., FASEB J., 10, 1213-1218, 1996). An increase in glucose exposure is coupled through GK in β-cells to increased insulin secretion and in hepatocytes to increased glycogen deposition and perhaps decreased glucose production.

The fmding that type II maturity-onset diabetes of the young (MODY-2) is caused by loss of function mutations in the GK gene suggests that GK also functions as a glucose sensor in humans (Liang, Y., Kesavan, P., Wang, L. et al., Biochem. J. 309, 167-173, 1995). Additional evidence supporting an important role for GK in the regulation of glucose metabolism in humans was provided by the identification of patients that express a mutant form of GK with increased enzymatic activity. These patients exhibit a fasting hypoglycemia associated with an inappropriately elevated level of plasma insulin (Glaser, B., Kesavan, P., Heyman, M. et al., New England J. Med. 338, 226-230, 1998). While mutations of the GK gene are not found in the majority of patients with type II diabetes, compounds that activate GK and, thereby, increase the sensitivity of the GK sensor system will still be useful in the treatment of the hyperglycemia characteristic of all type II diabetes. Glucokinase activators will increase the flux of glucose metabolism in β-cells and hepatocytes, which will be coupled to increased insulin secretion. Such agents would be useful for treating type II diabetes.

In an embodiment of the present invention, provided is a compound of formula (I): 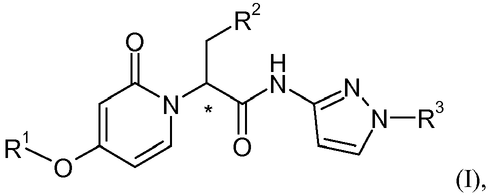 wherein
- R¹ is: aryl, said aryl being unsubstituted or mono-, bi- or tri-substituted independently with halogen, phenyl, lower alkyl, lower halogenalkyl, lower alkoxy or lower halogenalkoxy, or
heteroaryl, said heteroaryl being unsubstituted or substituted with halogen or lower alkyl, or
- R² is: selected from the group consisting of lower alkyl, lower halogenalkyl, and cycloalkyl, said cycloalkyl being unsubstituted or substituted by halogen, oxo or hydroxy; and
- R³ is: lower alkyl or lower halogenalkyl;
or pharmaceutically acceptable salts thereof.

* denotes an asymmetric C-atom.

In a still yet another preferred embodiment of the present invention, provided is a pharmaceutical composition, comprising a therapeutically effective amount of a compound according to formula (I) or a pharmaceutically acceptable salt thereof and a pharmaceutically acceptable carrier and/or adjuvant.

In another preferred embodiment, R¹ is aryl, said aryl being unsubstituted or mono-, bi- or tri-substituted independently with halogen, phenyl, lower alkyl or alkoxy.

In a further preferred embodiment, R¹ is aryl selected from phenyl or naphthalen-1-yl.

In another preferred embodiment, R¹ is phenyl, unsubstituted or mono-, bi- or tri-substituted independently with halogen, phenyl, lower alkyl, lower halogenalkyl, lower alkoxy or lower halogenalkoxy. In another preferred embodiment, R¹ is phenyl, unsubstituted or mono-, bi- or tri-substituted with fluorine, chlorine, phenyl, -CF₃, -CH₃, -OCF₃, -OCH₃ or -CH(CH₃)₂.

In another preferred embodiment, R¹ is phenyl bi-substituted with fluorine.

In another preferred embodiment, R¹ is phenyl substituted with both chlorine and -CH₃.

In another preferred embodiment, R¹ is naphthalen-1-yl.

In another preferred embodiment, R¹ is pyridyl, unsubstituted or substituted with halogen or lower alkyl.

In another preferred embodiment, R¹ is unsubstituted pyridine.

In another preferred embodiment, R² is -CH(CH₃)₂.

In another preferred embodiment, R³ is lower alkyl, more preferably methyl.

In a further embodiment of the present invention, provided is a compound of formula (I), wherein
- R¹ is: -aryl, unsubstituted or mono-, bi- or tri-substituted independently with halogen, phenyl, lower alkyl or alkoxy,
-heteroaryl, unsubstituted or substituted with halogen or lower alkyl, or -naphthalen-1-yl;
- R² is: -lower alkyl; and
- R³ is: -lower alkyl;
or pharmaceutically acceptable salts thereof.

In another preferred embodiment, the compound according to formula (I) is selected from the group consisting of:
2-[4-(2,3-difluoro-phenoxy)-2-oxo-2H-pyridin-1-yl]-4-methyl-pentanoic acid (1-methyl-1H-pyrazol-3-yl)-amide;
2-[4-(3-fluoro-phenoxy)-2-oxo-2H-pyridin-1-yl]-4-methyl-pentanoic acid (1-methyl-1H-pyrazol-3-yl)-amide;
4-methyl-2-(2-oxo-4-phenoxy-2H-pyridin-1-yl)-pentanoic acid (1-methyl-1H-pyrazol-3-yl)-amide;
4-methyl-2-(2-oxo-4-o-tolyloxy-2H-pyridin-1-yl)-pentanoic acid (1-methyl-1H-pyrazol-3-yl)-amide;
2-[4-(2,6-difluoro-phenoxy)-2-oxo-2H-pyridin-1-yl]-4-methyl-pentanoic acid (1-methyl-1H-pyrazol-3-yl)-amide;
4-methyl-2-[2-oxo-4-(2-trifluoromethyl-phenoxy)-2H-pyridin-1-yl]-pentanoic acid (1-methyl-1H-pyrazol-3-yl)-amide;
2-[4-(4-fluoro-phenoxy)-2-oxo-2H-pyridin-1-yl]-4-methyl-pentanoic acid (1-methyl-1H-pyrazol-3-yl)-amide;
4-methyl-2-[2-oxo-4-(pyridin-3-yloxy)-2H-pyridin-1-yl]-pentanoic acid (1-methyl-1H-pyrazol-3-yl)-amide;
2-[4-(biphenyl-2-yloxy)-2-oxo-2H-pyridin-1-yl]-4-methyl-pentanoic acid (1-methyl-1H-pyrazol-3-yl)-amide;
2-[4-(2-isopropyl-phenoxy)-2-oxo-2H-pyridin-1-yl]-4-methyl-pentanoic acid (1-methyl-1H-pyrazol-3-yl)-amide;
2-[4-(2,4-difluoro-phenoxy)-2-oxo-2H-pyridin-1-yl]-4-methyl-pentanoic acid (1-methyl-1H-pyrazol-3-yl)-amide;
2-[4-(3-chloro-2-methyl-phenoxy)-2-oxo-2H-pyridin-1-yl]-4-methyl-pentanoic acid (1-methyl-1H-pyrazol-3-yl)-amide;
2-[4-(2,5-difluoro-phenoxy)-2-oxo-2H-pyridin-1-yl]-4-methyl-pentanoic acid (1-methyl-1H-pyrazol-3-yl)-amide;
4-methyl-2-[4-(naphthalen-1-yloxy)-2-oxo-2H-pyridin-1-yl]-pentanoic acid (1-methyl-1H-pyrazol-3-yl)-amide;
2-[4-(2-chloro-phenoxy)-2-oxo-2H-pyridin-1-yl]-4-methyl-pentanoic acid (1-methyl-1H-pyrazol-3-yl)-amide;
4-methyl-2-[2-oxo-4-(2-trifluoromethoxy-phenoxy)-2H-pyridin-1-yl]-pentanoic acid (1-methyl-1 H-pyrazol-3-yl)-amide;
2-[4-(2-fluoro-phenoxy)-2-oxo-2H-pyridin-1-yl]-4-methyl-pentanoic acid (1-methyl-1H-pyrazol-3-yl)-amide; or
2-[4-(2-methoxy-phenoxy)-2-oxo-2H-pyridin-1-yl]-4-methyl-pentanoic acid (1-methyl-1H-pyrazol-3-yl)-amide.

In a still yet another preferred embodiment of the present invention, provided are pharmaceutical compositions, comprising a therapeutically effective amount of a compound according to formula (I) or a pharmaceutically acceptable salt thereof and a pharmaceutically acceptable carrier.

In a further embodiment, the invention is concerned with a compound of formula (I) for use as therapeutic active substance, preferably for use in treating a metabolic disease and/or disorder, more preferably, for use in the treatment of diabetes mellitus.

It is to be understood that the terminology employed herein is for the purpose of describing particular embodiments, and is not intended to be limiting. Further, although any methods, devices and materials similar or equivalent to those described herein can be used in the practice or testing of the invention, the preferred methods, devices and materials are now described.

As used herein, the term "alkyl", alone or in combination with other groups, refers to a branched or straight-chain monovalent saturated aliphatic hydrocarbon radical of one to twenty carbon atoms, preferably one to sixteen carbon atoms, more preferably one to ten carbon atoms.

The term "lower alkyl", alone or in combination with other groups, refers to a branched or straight-chain alkyl radical of one to nine carbon atoms, preferably one to six carbon atoms. This term is further exemplified by radicals such as methyl, ethyl, n-propyl, isopropyl, n-butyl, s-butyl, isobutyl, t-butyl, n-pentyl, 3-methylbutyl, n-hexyl, 2-ethylbutyl and the like, in particular methyl or isopropyl (-CH(CH₃)₂).

The term "cycloalkyl" refers to a monovalent mono- or polycarbocyclic radical of three to ten, preferably three to seven carbon atoms and more preferably four to six carbon atoms. This term is further exemplified by radicals such as cyclopropyl, cyclobutyl, cyclopentyl, cyclohexyl, cycloheptyl, bornyl, adamantyl, indenyl and the like. In a preferred embodiment, "cycloalkyl" means cyclobutyl, cyclopentyl or cyclohexyl.

The term "aryl" refers to an aromatic mono- or polycarbocyclic radical of 6 to 12 carbon atoms having at least one aromatic ring. Examples of such groups include, but are not limited to, phenyl or naphthyl. The term also includes partially saturated groups such as 1,2,3,4-tetrahydronaphthalene, 1,2-dihydronaphthalene, indanyl, 1H-indenyl and the like.

The term "heteroaryl" refers to an aromatic mono- or polycyclic radical of 5 to 12 atoms having at least one aromatic ring containing one, two, or three ring heteroatoms selected from N, O, and S, with the remaining ring atoms being C. One or two ring carbon atoms of the heteroaryl group may be replaced with a carbonyl group. Examples of "heteroaryl" groups are pyrrolyl, pyrazolyl, imidazolyl, triazolyl, tetrazolyl, oxazolyl, oxadiazolyl, isoxazolyl, thiadiazolyl, thiazolyl, furanyl, thienyl, pyridyl, pyrazinyl, pyrimidinyl, pyridazinyl, indolyl, isoindolyl, indazolyl, 7-azaindolyl, quinolinyl, isoquinolinyl, naphtyridyl, cinnolinyl, pyrazolo[1,5-a]pyridyl, imidazo[1,2-a]pyridyl, quinoxalinyl, benzofuranyl, benzoxazinyl, benzothiazolyl, benzotriazolyl, chromenyl, chromanyl, isochromanyl, coumarinyl, isocoumarinyl and benzopyranyl. Particular heteroaryl groups are selected from the group consisting of pyridyl, pyrazinyl and pyrimidinyl. More particularly, "heteroaryl" means pyridyl.

As used herein, the term "lower alkoxy" means the group R'-O-, wherein R' is lower alkyl and the term "lower alkyl" has the previously given significance. Examples of lower alkoxy groups are methoxy, ethoxy, n-propoxy, isopropoxy, n-butoxy, isobutoxy, sec.-butoxy and tert.-butoxy, preferably methoxy and ethoxy.

As used herein, the term "halogen" means a fluorine, chlorine, bromine or iodine radical, preferably a fluorine, chlorine or bromine radical, and more preferably a fluorine or chlorine radical.

The term "lower halogenalkyl" refers to lower alkyl groups as defmed above wherein at least one of the hydrogen atoms of the lower alkyl group is replaced by a halogen atom, preferably fluoro or chloro, most preferably fluoro. Among the preferred halogenated lower alkyl groups are trifluoromethyl, difluoromethyl, trifluoroethyl, 2,2-difluoroethyl, fluoromethyl and chloromethyl, with trifluoromethyl being especially preferred.

The term "lower halogenalkoxy" means lower alkoxy groups as defmed above wherein at least one of the hydrogen atoms of the lower alkoxy group is replaced by a halogen atom, preferably fluoro or chloro, most preferably fluoro. Among the preferred halogenated lower alkoxy groups are trifluoromethoxy, difluoromethoxy, fluormethoxy and chloromethoxy, with trifluoromethoxy being especially preferred.

Compounds of formula (I) can have one or more asymmetric carbon atoms and can exist in the form of optically pure enantiomers, mixtures of enantiomers such as, for example, racemates, optically pure diastereoisomers, mixtures of diastereoisomers, diastereoisomeric racemates or mixtures of diastereoisomeric racemates. The optically active forms can be obtained for example by resolution of the racemates, by asymmetric synthesis or asymmetric chromatography (chromatography with chiral adsorbents or eluant). The invention embraces all of these forms.

As used herein, the term "pharmaceutically acceptable salt" means any pharmaceutically acceptable salt of the compound of formula (I). Salts may be prepared from pharmaceutically acceptable non-toxic acids and bases including inorganic and organic acids and bases. Such acids include, for example, acetic, benzenesulfonic, benzoic, camphorsulfonic, citric, ethenesulfonic, dichloroacetic, formic, fumaric, gluconic, glutamic, hippuric, hydrobromic, hydrochloric, isethionic, lactic, maleic, malic, mandelic, methanesulfonic, mucic, nitric, oxalic, pamoic, pantothenic, phosphoric, succinic, sulfuric, tartaric, oxalic, p-toluenesulfonic and the like. Particularly preferred are fumaric, hydrochloric, hydrobromic, phosphoric, succinic, sulfuric and methanesulfonic acids. Acceptable base salts include alkali metal (e.g. sodium, potassium), alkaline earth metal (e.g. calcium, magnesium) and aluminium salts.

In the practice of the method of the present invention, an effective amount of any one of the compounds of this invention or a combination of any of the compounds of this invention or a pharmaceutically acceptable salt thereof, is administered via any of the usual and acceptable methods known in the art, either singly or in combination. The compounds or compositions can thus be administered orally (e.g., buccal cavity), sublingually, parenterally (e.g., intramuscularly, intravenously, or subcutaneously), rectally (e.g., by suppositories or washings), transdermally (e.g., skin electroporation) or by inhalation (e.g., by aerosol), and in the form or solid, liquid or gaseous dosages, including tablets and suspensions. The administration can be conducted in a single unit dosage form with continuous therapy or in a single dose therapy ad libitum. The therapeutic composition can also be in the form of an oil emulsion or dispersion in conjunction with a lipophilic salt such as pamoic acid, or in the form of a biodegradable sustained-release composition for subcutaneous or intramuscular administration.

Useful pharmaceutical carriers for the preparation of the compositions hereof, can be solids, liquids or gases; thus, the compositions can take the form of tablets, pills, capsules, suppositories, powders, enterically coated or other protected formulations (e.g. binding on ion-exchange resins or packaging in lipid-protein vesicles), sustained release formulations, solutions, suspensions, elixirs, aerosols, and the like. The carrier can be selected from the various oils including those of petroleum, animal, vegetable or synthetic origin, e.g., peanut oil, soybean oil, mineral oil, sesame oil, and the like. Water, saline, aqueous dextrose, and glycols are preferred liquid carriers, particularly (when isotonic with the blood) for injectable solutions. For example, formulations for intravenous administration comprise sterile aqueous solutions of the active ingredient(s) which are prepared by dissolving solid active ingredient(s) in water to produce an aqueous solution, and rendering the solution sterile. Suitable pharmaceutical excipients include starch, cellulose, talc, glucose, lactose, talc, gelatin, malt, rice, flour, chalk, silica, magnesium stearate, sodium stearate, glycerol monostearate, sodium chloride, dried skim milk, glycerol, propylene glycol, water, ethanol, and the like. The compositions may be subjected to conventional pharmaceutical additives such as preservatives, stabilizing agents, wetting or emulsifying agents, salts for adjusting osmotic pressure, buffers and the like. Suitable pharmaceutical carriers and their formulation are described in Remington's Pharmaceutical Sciences by E. W. Martin. Such compositions will, in any event, contain an effective amount of the active compound together with a suitable carrier so as to prepare the proper dosage form for proper administration to the recipient.

The dose of a compound of the present invention depends on a number of factors, such as, for example, the manner of administration, the age and the body weight of the subject, and the condition of the subject to be treated, and ultimately will be decided by the attending physician or veterinarian. Such an amount of the active compound as determined by the attending physician or veterinarian is referred to herein, and in the claims, as a "therapeutically effective amount". For example, the dose of a compound of the present invention is typically in the range of about 1 to about 1000 mg per day. Preferably, the therapeutically effective amount is in an amount of from about 1 mg to about 500 mg per day.

It will be appreciated, that the compounds of general formula (I) in this invention may be derivatized at functional groups to provide derivatives which are capable of conversion back to the parent compound *in vivo.* Physiologically acceptable and metabolically labile derivatives, which are capable of producing the parent compounds of general formula I *in vivo* are also within the scope of this invention.

Compounds of the present invention can be prepared beginning with commercially available starting materials and utilizing general synthetic techniques and procedures known to those skilled in the art. Outlined below are reaction schemes suitable for preparing such compounds. Chemicals may be purchased from companies such as for example Aldrich, Argonaut Technologies, VWR and Lancaster. Chromatography supplies and equipment may be purchased from such companies as for example AnaLogix, Inc, Burlington, WI; Biotage AB, Charlottesville, VA; Analytical Sales and Services, Inc., Pompton Plains, NJ; Teledyne Isco, Lincoln, NE; VWR International, Bridgeport, NJ; Varian Inc., Palo Alto, CA, and Multigram II Mettler Toledo Instrument Newark, DE. Biotage, ISCO and Analogix columns are pre-packed silica gel columns used in standard chromatography.

Preferably, the compounds of formula I can be prepared by the following General Reaction Scheme I.

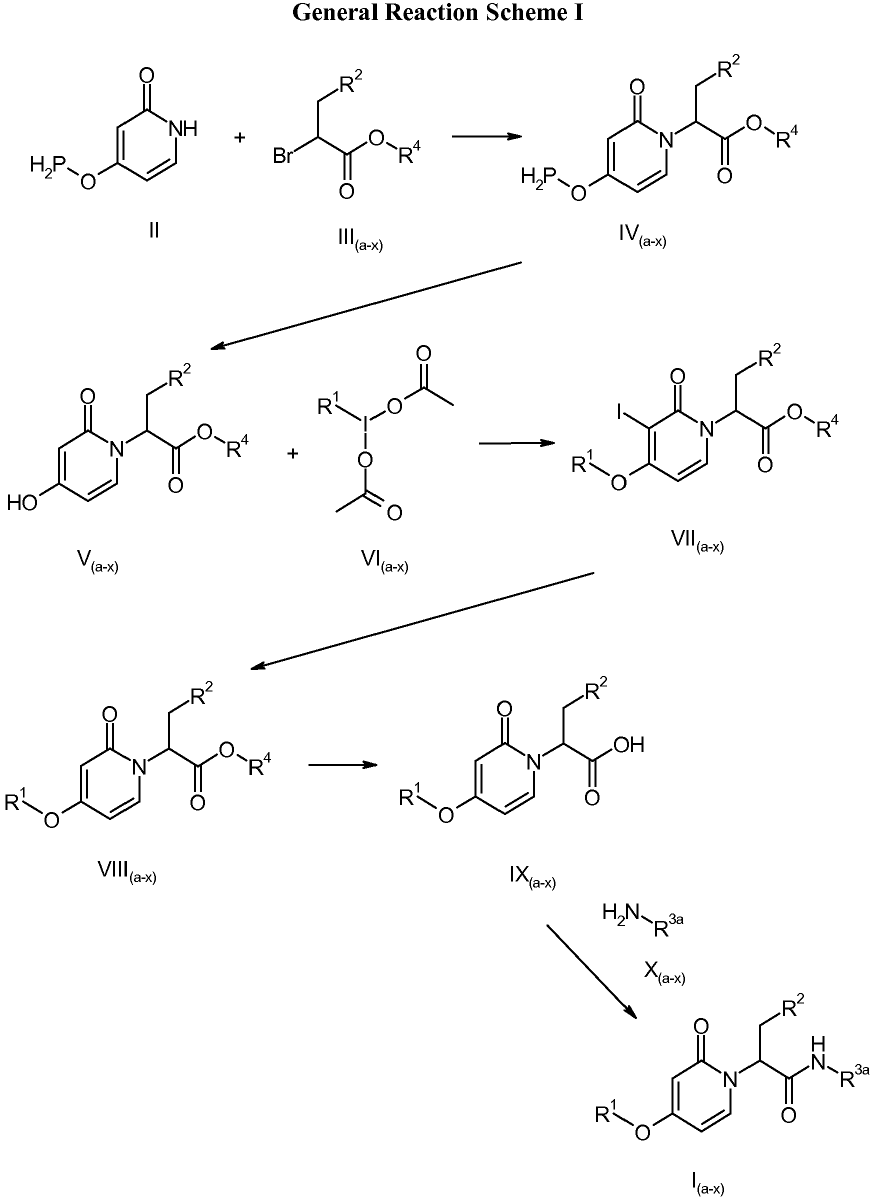

The compound of formula II where P is a benzyl protecting group is readily available from commercial sources or can be prepared from commercially available 2,4-dihydroxypyridine (see for example, *PCT Int. Appl.* WO2008/022979 A1).

A number of amino acids are also available from commercial sources. Where not commercially available, amino acids can be prepared using literature methods.

The compounds of formula III may be prepared from amino acids and protected amino acids. The compounds of formula III may be prepared wherein R² is aryl, substituted aryl, heteroaryl, substituted heteroaryl, lower alkyl, lower halogenalkyl, cycloalkyl, substituted cycloalkyl, heterocyclyl, or substituted heterocyclyl. When these compounds are available from commercially available sources, the appropriate protected or unprotected amino acid may be converted to the desired halo ester, where bromide is the preferred halogen, through conventional methods. An example of a method to convert an amino group to a halogen, preferably bromide utilizes the formation of a diazonium species which can then be converted in *situ* to a halogen, preferably bromide (see for example, Archer, C. H., Thomas, N. R., Gani, D. Tet. Asymm., 1993, 4(6), 1141-1152; Dener, J. M., Zhang, L.-H., Rapoport, H. J. Org. Chem., 1993, 58, 1159-1166; Souers, A. J., Schurer, S., Kwack, H., Virgilio, A. A., Ellman, J. A, Synthesis, 1999, 4, 583-585). The resulting halo-acid may either be maintained as the acid or may then be converted to an appropriately functionalized ester or amide by any conventional method of converting an acid to an ester or an amide (see for example, Archer, C. H., Thomas, N. R., Gani, D. Tet. Asymm., 1993, 4(6), 1141-1152; *PCT Int. Appl.* WO 03/055482 A1).

The compounds of formula III where R² is aryl, substituted aryl, heteroaryl, substituted heteroaryl, lower alkyl, lower halogenalkyl, cycloalkyl, substituted cycloalkyl, heterocyclyl, or substituted heterocyclyl may be produced from commercially available material (see for example, U.S. Patent No. 4,977,144). For example, the appropriate R² derivative may be reacted with a malonate derivative under standard conditions to produce a substituted malonate (see for example, Kortylewicz, Z.P., Galardy, R.E., J. Med. Chem., 1990, 33, 263-273). The resulting substituted malonate may then be treated under hydrolysis conditions to form the resulting diacid (see for example, Kortylewicz, Z.P., Galardy, R.E., J. Med. Chem., 1990, 33, 263-273). The diacid may then be heated under such conditions that will promote a decarboxylation to form the appropriately substituted acid (see for example, Kortylewicz, Z.P., Galardy, R.E., J. Med. Chem. 1990, 33, 263-273). In some instances, the desired mono-acid is available from commercial sources. The resulting substituted acid can then be treated under conditions that may form an acid chloride (see for example, Epstein, J.W., Brabander, H.J., Fanshawe, W.J., Hofmann, C.M., McKenzie, T.C., Safir, S.R., Osterberg, A.C., Cosulich, D.B., Lovell, F.M., J. Med.Chem., 1981, 24, 481-490). The remaining acid chloride can then be treated with a hydroxyl containing reagent, such as methanol, to form the corresponding compound of formula III.

For the compounds of formula III in cases where R₂ is aryl, substituted aryl, heteroaryl, substituted heteroaryl, lower alkyl, lower halogenalkyl, cycloalkyl, substituted cycloalkyl, heterocyclyl, or substituted heterocyclyl and the amino acid or functionalized version thereof is not available from commercial sources, the amino acid may be produced if desired through conventional methods. Several natural and unnatural amino acids are commercially available or readily available via several methods reported in the literature (see reviews, for e.g. D. J. Ager, in Handbook of chiral chemicals, 2nd Edition, p 11-30, CRC Press). Among these methods are asymmetric hydrogenation of the enamides (see for example Ager, D.J., Laneman, S. A., The Synthesis of Unnatural Amino Acids, in Asymmetric Catalysis on Industrial Scale, Blaser, H. -U., Schmidt, E.,, Wiley-VCH: Weinheim, 2004, p 23), chiral auxiliary derived asymmetric induction methods (see for example Schollkopf, U. Pure and App. Chem. 1983, 55, 1799-1806; Oppolzer, W.; Moretti, R. Tetrahedron, 1988, 44, 5541; Evans, D. A.; Britton, T. C.; Ellman, J. A.; Dorow, R. L. J. Amer. Chem. Soc., 1990, 112, p4011) and asymmetric methods using chiral phase transfer catalyzed alkylations (see for example O'Donnell, M. J., Acc. Chem. Research 2004, 37, 506). Using these methods compounds of formula III, where R₂ is alkyl, cycloalkyl, haloalkyl, heterocycloalkyl, aryl or heteroaryl groups can be prepared.

The alkyl and cycloalkyl amino acids such as, cyclopentyl alanine, cyclohexyl alanine, and cyclobutyl alanine are either commercially available or are readily available from corresponding halides or tosylates or mesylates via the general methods described above. Similarly, aryl and heteroaryl containing amino acids are either commercially available or can be prepared from readily accessible aryl or heteroaryl methyl halides, using the standard methods, described before. Amino acids such as, 2,6-fluorophenyl alanine, 2-thienyl alanine, 2-amino-3-isoxazol-5-yl-propionic acid can be prepared. Several fluoro- and chloro- substituted leucines, for example, 2-amino-4-fluoro-4-methyl-pentanoic acid, 2-amino-4-chloro-4-methyl-pentanoic acid, 2-amino-5,5,5-trifluoro-4-methyl-pentanoic acid, 2-amino-4,4-difluoro-butyric acid, 2-amino-4,4,4-trifluoro-butyric acid, and 2-amino-4,4-dichloro-butyric acid are readily accessible from known methods described in literature (Gauthier, J. Y. et al, Bioorg. & Med. Chem. Lett., 2008, 923-928). Hydroxy substituted leucine, 2-amino-4-hydroxy-4-methyl-pentanoic acid, can be prepared from appropriately substituted leucine, via its *N*-bromosuccinimide reaction, as reported (Easton, C. J. et al, Tetrahedron Lett., 1990, 131, 7059,) Similarly, fluoro- substituted cycloalkyl amino acids can be obtained via known methods (see for example, Qiu, X.-L.; Meng, W.-D.; Qing, F.-L., Tetrahedron, 2004, 60, 6711). If a gem- difluoro cycloalkyl is required, it can be obtained via the corresponding keto- derivative, using diethylaminosulfurtrifluoride (DAST) reagent (Middleton, W. J.; Bingham, E. M., Organic Syn., 1977, 57, 50; Haas, A.; Lieb, M., Chimia, 1985, 35, 134). Cycloalkanone containing amino acids, for example, cyclopentan-3-one, can be prepared using the appropriately protected cyclopentane-3-one methyl tosylate or mesylate (*PCT Int. Appl.* WO 2003095438; *PCT Int. Appl.* WO 2007115968) resulting in the preparation of protected amino acid, 2-amino-3-(8,8-dimethyl-6,10-dioxa-spiro[4.5]dec-2-yl)-propionic acid via the general methods of amino acid synthesis described above. Amino acid derivatives, with pyrrolidinone ring, 2-amino-3-(2-oxo-pyrrolidin-3-yl)-propionic acid methyl ester can be prepared using literature reports (Ramsamy, K.; Olsen, R. K.; Emery, T., Synthesis, 1982,1, 42-43, Eustache, J.; Grob, A.; Lam, C.; Sellier, O.; Schulz, G. Bioorg. Med. Chem. Lett., 1998 8, 2961-2966). Heterocyclyl containing amino acid, is commercially available, 2-amino-3-(tetrahydro-pyran-4-yl)-propionic acid, while the corresponding analog, 2-amino-3-(tetrahydro-pyran-2-yl)-propionic acid can be prepared using reported procedures *(PCT Int. Appl.* WO2001005783; *PCT Int. Appl.* W02007070201). The amino acids with 2-tetrahydrofuran ring, 2-amino-3-(tetrahydro-furan-2-yl)-propionic acid can be prepared from the 2-furyl derivative via the hydrogenation of 2-furyl ring and subsequent diastereomer separation using standard methods (see for example, *PCT Int. Appl.* WO 2004033462; *PCT Int. Appl.* W09214706). Amino acids with bicyclic systems like norbornyl rings are readily accessible. For example commercially available 2-norborananemethanol, which can be converted to the amino acid derivative using standard methods described above.

For amino acid derivatives of Formula III where R² is cycloalkyl substituted with a fluorine on the methine ring attachment carbon atom, such as 2-amino-3-(1-fluoro-cyclobutyl)-propionic acid, 2-amino-3-(1-fluoro-cyclopentyl)-propionic acid, or 2-amino-3-(1-fluoro-cyclohexyl)-propionic acid. These compounds can be prepared by alkylating (benzhydrylidene-amino)-acetic acid alkyl esters with triflate, tosylate or mesylate derivatives of the corresponding (1-fluoro-cycloalkyl)-methanol analogs or the corresponding bromides. The resulting benzhydrylidene derivatives can be converted to the amino acids using standard procedures (see for example Venkatraman, S.; Bogen, S. L.; Arasappan, A.; Bennett, F.; Chen, K.; Jao, E.; Liu, Y.-T.; Lovey, R.; Hendrata, S.; Huang, Y.; Pan, W.; et al.; J. Med. Chem.; 2006 49, 6074 - 6086) The triflate, tosylate or mesylate derivatives can be prepared from the alcohols using any conditions known for converting an alcohol to a triflate, tosylate or mesylate. The bromide derivatives can be prepared from the alcohols using any conditions known for converting an alcohol to a bromide. The (1-fluoro-cycloalkyl)-methanol analogs are known in the literature (see for example; Mongelli, N.; Animati, F.; D'Alessio, R.; Zuliani, L.; Gandolfi, C.. Synthesis 1988, 4, 310-13.; *PCT Int. Appl.* WO 2006064286) or can be prepared from the corresponding epoxide (Demjanow; D. Chem. Ber. 1922, 55, 2725) by treatment with an appropriate fluorinating reagent, for example pyridine-hydroflouride (see for example Haufe, G.; Wessel, U.; Schulze, K; Alvemhe, G.; J. Fluorine Chem.; 1995; 74; 283-292.)

For amino acid derivatives of Formula III where R² is alkyl or cycloalkyl substituted with a hydroxyl group on the methine ring attachment carbon atom, such as 2-amino-4-hydroxy-4-methyl-pentanoic acid, 2-amino-3-(1-hydroxy-cyclobutyl)-propionic acid, 2-amino-3-(1-hydroxy-cyclopentyl)-propionic acid, or 2-amino-3-(1-hydroxy-cyclohexyl)-propionic acid. These compounds can be prepared by alkylating (benzhydrylidene-amino)-acetic acid alkyl esters with triflate, tosylate or mesylate derivatives of the corresponding (1-hydroxy-cycloalkyl)-methanol analogs (1-hydroxymethyl-cyclohexanol is commercially available; for 2-methyl-propane-1,2-diol see Richardson, W. H. J. Org. Chem. 1989, 54, 4677-4684.; Richardson, W. H.; Lovett, M. B.; Olson, L. J. Org. Chem 1989, 54, 3523-3525., for 1-hydroxymethyl-cyclopentanol see Tamao, K.; Ishida, N. Tetrahedron Lett. 1984, 25, 4245-4248, for 1-hydroxymethyl-cyclobutanol see Roberts, J. D.; Sauer, C. W. J. Am. Chem. Soc. 1949, 71, 3925-3929; Wade, P. A.; Kondracki, P. A. J. Org. Chem. 1993, 58, 3140-3147), corresponding bromides (for 1-halo-2-methyl-propan-2-ol see Mueller, D. C.; Seyferth, D. Organometal. Chem. Syn. 1971, 1, 127-144., for 1-halomethyl-cyclopentanol see Traynham, J. G.; Pascual, O. S. Tetrahedron 1959, 7, 165-172; Okabe, M.; Tada, M. Bull. Chem. Soc. Jpn 1982, 55, 1498-1503; Baumstark, A. L.; Niroomand, F.; Vasquez, P. C. J. Org. Chem. 1984, 49, 4497-4500; Tabuchi, T.; Inanaga, J.; Yamaguchi, M. Tetrahedron Lett. 1986, 27, 3891-3894; Canonne, P.; Belley, M.; Fytas, G.; Plamondon, J. Can. J Chem. 1988, 66, 168-173.; Jereb, M.; Zupan, M.; Stavber, S. Green Chem. 2005, 7, 100-104, for 1-halomethyl-cyclobutanol see Traynham, J. G.; Pascual, O. S. Tetrahedron 1959, 7, 165-172; Erickson, K. L.; Kim, K. J. Org. Chem 1971, 36, 2915-2916; Erickson, K. L. J. Org. Chem. 1973, 38, 1463-1469, for 1-halomethyl-cyclohexanol see Detty, M. R. J. Org. Chem 1980, 45, 924-926.; Detty, M. R.; Seidler, M. D. J. Org. Chem. 1981, 46, 1283-1292; Baumstark, A. L.; Niroomand, F.; Vasquez, P. C. J. Org. Chem. 1984, 49, 4497-4500), or corresponding tertiary alcohol protected analogs (for 1-hydroxy-2-methyl-propan-2-ol see Denmark, S. E.; Stavenger, R. A. J. Am. Chem. Soc. 2000, 122, 8837-8847, for 1-hydroxymethyl-cyclopentanol see *PCT Inter. Appl.* WO19960117, for 1-hydroxymethyl-cyclohexanol see Tanino, K.; Shimizu, T.; Kuwahara, M.; Kuwajima, I. J. Org. Chem. 1998, 63, 2422-2423). The resulting benzhydrylidene derivatives can be converted to the amino acids using standard procedures (see for example Venkatraman, S.; Bogen, S. L.; Arasappan, A.; Bennett, F.; Chen, K.; Jao, E.; Liu, Y.-T.; Lovey, R.; Hendrata, S.; Huang, Y.; Pan, W.; et al.; J. Med. Chem.; 2006 49, 6074 - 6086) The triflate, tosylate or mesylate derivatives can be prepared from the alcohols using any conditions known for converting an alcohol to a triflate, tosylate or mesylate. The bromide derivatives can be prepared from the alcohols using any conditions known for converting an alcohol to a bromide. Alternatively these compounds can be prepared by condensing the corresponding aldehydes with glycine, protected glycine or protected glycine phosphonate derivatives followed by hydrogenation (see for example Ojima, I.; Kato, K.; Nakahashi, K.; Fuchikami, T.; Fujita, M. J. Org. Chem. 1989, 54, 4511-4522 Alexander, P. A.; Marsden, S. P.; Munoz Subtil, D. M.; Reader, J. C. Org. Lett. 2005, 7, 5433-5436; Davies, J. R.; Kane, P. D.; Moody, C. J.; Slawin, A. M. Z. J. Org. Chem. 2005, 70, 5840-5851). The corresponding alcohol protected aldehydes are known in the literature (for protected 2-hydroxy-2-methyl-propionaldehyde see Denmark, S. E.; Stavenger, R. A. J. Am. Chem. Soc. 2000, 122, 8837-8847; Frezza, M.; Soulere, L.; Queneau, Y.; Doutheau, A. Tetrahedron Lett. 2005, 46, 6495-6498; Trost, B. M.; Shin, S.; Sclafani, J. A. J. Am. Chem. Soc. 2005, 127, 8602-8603, for protected 1-hydroxy-cyclopentanecarbaldehyde see Parkes, K. E. B.; Pattenden, G. J. Chem. Soc., Perkin Trans. 11988, 1119-1134, for protected 1-hydroxy-cyclohexanecarbaldehyde see Ito, Y.; Matsuura, T.; Murakami, M. J. Am. Chem. Soc. 1987, 109, 7888-7890; Matsuda, T.; Tanino, K.; Kuwajima, I. Tetrahedron Lett. 1989, 30, 4267-4270; Hayashi, M.; Yoshiga, T.; Oguni, N. Synlett 1991, 479-480; Hayashi, M.; Yoshiga, T.; Nakatani, K.; Ono, K.; Oguni, N. Tetrahedron 1994, 50, 2821-2830; Tanino, K.; Shimizu, T.; Kuwahara, M.; Kuwajima, I. J. Org. Chem. 1998, 63, 2422-2423) or can be prepared form the alcohols using any method suitable for oxidizing a primary alcohol to an aldehyde. Unmasking of the alcohol functionality can be accomplished using any conditions known for converting a protected alcohol such as a silyl protected alcohol or an ester protected alcohol to an alcohol.

For amino acid derivatives of Formula III where R² is a geminal dihalogenalkyl group such as 2-amino-4,4-difluoro-butyric acid, 2-amino-4,4-dichloro-butyric acid or 2-amino-4,4-difluoro-pentanoic acid, these compounds, or their suitably protected derivatives, can be prepared as described in the literature (*PCT Int. Appl.* WO 2005040142, Synthesis 1996, 12, 1419-1421).

The compounds of formula IV may be produced from the compounds of formula III and the compounds of formula II through standard methods that are described in the literature (see for example, *PCT Inter. Appl.* WO 2003/068230 A1).

The compounds of formula V may be produced from the compounds of formula IV through standard methods to remove oxygen linked protecting groups (see for example, *PCT Inter. Appl.* WO 2003/068230 A1; Greene, T. W. Protective Groups in Organic Synthesis; John Wiley & Sons, Inc.: New York, 1991).

The compound of Formula VI may be produced from commercially available starting material using standard conditions. (see for example, Molecules, 2005, 10, 190-194). More preferably the following reagents, which are all commercially available, can be used: 1,2-difluoro-3-iodo-benzene, 1-fluoro-3-iodo-benzene, iodo-benzene, 1-iodo-2-methyl-benzene, 1,3-difluoro-2-iodo-benzene, 1-iodo-2-trifluoromethyl-benzene, 1-fluoro-4-iodo-benzene, 3-iodo-pyridine, 2-iodo-biphenyl, 1-iodo-2-isopropyl-benzene, 2,4-difluoro-1-iodo-benzene, 1-chloro-3-iodo-2-methyl-benzene, 1,4-difluoro-2-iodo-benzene, 1-iodo-naphthalene, 1-chloro-2-iodo-benzene, 1-iodo-2-trifluoromethoxy-benzene, 1-fluoro-2-iodo-benzene, 1-iodo-2-methoxy-benzene.

The compounds of formula VII may be produced from the compounds of formula V and the compounds of formula VI using standard conditions (see for example, Bioorg. Med. Chem. Lett., 2007, 17, 712-716; Bioorg. Med. Chem. Lett., 2003, 13, 4309-4312).

The compounds of formula VIII may be produced from the compounds of formula VII using standard reduction conditions (see for example, Anorganische Chemie, 1983, 38, 398 - 403).

The compounds of formula IX may be produced from the compounds of formula VIII. For the compounds of formula VIII, R⁴ may be an alkyl or any substituent that may be removed through conventional methods to convert an ester to a carboxylic acid, preferably via hydrolysis (see for example, New, J.S., Christopher, W.L., Jass, P.A., J. Org. Chem., 1989, 54, 990-992).

Compounds of formula X may be unsubstituted or substituted heteroaryl or heterocyclylalkyl groups which are commercially available or known in the literature. More preferred heteroaryl groups include 2*H*-[1,2,3]triazol-4-yl, 1*H*-indol-7-yl, 5*H*-carbazol-1-yl, 2,3-dihydro-1*H*-indol-7-yl, 1*H*-pyrrolo[2,3-c]pyridin-7-yl, 4,5,6,6a-tetrahydro-3α*H-*cyclopenta[b]thiophen-2-yl, 2*H*-[1,2,4]triazol-3-yl, pyrimidin-4-yl, furazan-3-yl, pyridazin-3-yl, (Z)-4,6,8,10-tetrathia-5,7,9,11-tetraaza-cyclopentacyclodecen-5-yl, thiazol-4-yl, dihydro-1*H-*[1,2,4]triazol-3-yl, isoxazol-5-yl, 1*H*-imidazol-2-yl, 1*H*-benzoimidazol-2-yl, [1,2,5]thiadiazol-3-yl, oxazol-2-yl, benzooxazol-2-yl, 4,5-dihydro-oxazol-2-yl, pyrimidin-2-yl, [1,2,4]oxadiazol-5-yl, isoxazol-3-yl, [1,2,4]triazin-3-yl, [1,2,4]triazolo[1,5-a]pyridin-2-yl, 1*H*-indazol-3-yl, isoquinolin-3-yl, and quinolin-2-yl. Most preferred heteroaryl groups include 1*H*-pyrazol-3-yl, pyrazin-2-yl, pyridin-2-yl, thiazol-2-yl, [1,3,4]thiadiazol-2-yl, and [1,2,4]thiadiazol-5-yl.

If it is desired to produce the compound of formula X, where R^{3a} is a substituted 1*H-*pyrazol-3-yl group, most preferably: 1-acetyl-1*H*-pyrazol-3-yl, 1-*tert*-butoxycarbonyl-5-methyl-1*H*-pyrazol-3-yl, 1,5-dimethyl-1*H*-pyrazol-3-yl, or 5-methyl-1*H*-pyrazol-3-yl, these compounds are commercially available.

If it is desired to produce the compound of formula X, where R^{3a} is a substituted 1*H-*pyrazol-3-yl group, most preferably: 1-(2-*tert*-butoxycarbonylamino-ethyl)-1*H*-pyrazol-3-yl, 1-(2-isopropoxy-ethyl)-1*H*-pyrazol-3-yl, 1-(2-methoxy-2-methyl-propyl)-1*H*-pyrazol-3-yl, 1-(2-hydroxy-2-methyl-propyl)-1*H*-pyrazol-3-yl, 1-(2-hydroxy-propyl)-1*H*-pyrazol-3-yl, 1-(2-methyl-2-triethylsilanyloxy-propyl)-1*H*-pyrazol-3-yl, 1-(1-hydroxy-cyclopropylmethyl)-1*H-*pyrazol-3-yl, 1-(4-methoxycarbonyl-cyclohexylmethyl)-1*H*-pyrazol-3-yl, 1-2-(*tert*-butyl-dimethyl-silanyloxy)-ethyl-1*H*-pyrazol-3-yl, 1-(3-carboxy-benzyl)-1*H*-pyrazol-3-yl, 1-1-(4-methoxycarbonyl-phenyl)-butyl-1*H*-pyrazol-3-yl, 1-(3-*tert*-butoxycarbonylamino-benzyl)-1*H-*pyrazol-3-yl, 1-(3-methoxycarbonyl-benzyl)-1*H*-pyrazol-3-yl, 1-(4-*tert*-butoxycarbonylamino-but-2-ynyl)-1*H*-pyrazol-3-yl, 1-(4-hydroxy-but-2-ynyl)-1*H*-pyrazol-3-yl, 1-(3-methyl-but-2-enyl)-1*H*-pyrazol-3-yl, 1-(3-hydroxy-3-methyl-butyl)-1*H*-pyrazol-3-yl, 1-(4-methoxycarbonyl-benzyl)-1*H*-pyrazol-3-yl, 1-(3-methyl-butyl)-1*H*-pyrazol-3-yl, 1-isobutyl-1*H*-pyrazol-3-yl, 1-octyl-1*H*-pyrazol-3-yl, 1-hexyl-1*H*-pyrazol-3-yl, 1-(3-hydroxy-3-methyl-butyryl)-1*H*-pyrazol-3-yl, 1-((R)-2,3-dihydroxy-propyl)-1*H*-pyrazol-3-yl, 1-((S)-2,3-dihydroxy-propyl)-1*H*-pyrazol-3-yl, 1-ethanesulfonyl-1*H*-pyrazol-3-yl, 1-(4-methoxy-benzyl)-1*H*-pyrazol-3-yl, 1-(4-cyano-benzyl)-1*H*-pyrazol-3-yl, 1-(3-hydroxy-propyl)-1*H*-pyrazol-3-yl, 1-methanesulfonylmethyl-1*H-*pyrazol-3-yl, 1-(4-methanesulfonyl-benzyl)-1*H*-pyrazol-3-yl, 1-carbamoylmethyl-1*H*-pyrazol-3-yl, 1-(2-*tert*-butoxycarbonyl-ethyl)-1*H*-pyrazol-3-yl, 1-*tert*-butoxycarbonylmethyl-1*H*-pyrazol-3-yl, 1-propyl-1*H*-pyrazol-3-yl, 1-(4-chloro-benzyl)-1*H*-pyrazol-3-yl, 1-(2-methoxy-ethyl)-1*H-*pyrazol-3-yl, 1-cyclopropylmethyl-1*H-*pyrazol-3-yl, 1-(3,4-dichloro-benzyl)-1*H*-pyrazol-3-yl, 1-phenethyl-1*H*-pyrazol-3-yl, 1-*tert*-butoxycarbonyl-1*H*-pyrazol-3-yl, 1-isopropyl-1*H*-pyrazol-3-yl, 1-(4-methyl-benzyl)-1*H*-pyrazol-3-yl, 1-(4-hydroxy-butyl)-1*H-*pyrazol-3-yl, 1-butyl-1*H-*pyrazol-3-yl, 1-ethyl-1*H*-pyrazol-3-yl, 1-benzyl-1*H*-pyrazol-3-yl, 1-methyl-1*H*-pyrazol-3-yl, or 1*H*-pyrazol-3-yl, these compounds are commercially available or can be prepared as described in *U.S. Pat. Appl.* US 2008021032.

If it is desired to produce the compound of formula X, where R^{3a} is a substituted 1*H-*pyrazol-3-yl group, most preferably: 1-(dimethyl-phosphinoylmethyl)-1*H*-pyrazol-3-yl, 1-(diethoxy-phosphorylmethyl)-5-methyl-1*H*-pyrazol-3-yl, or 1-(diethoxy-phosphorylmethyl)-1*H-*pyrazol-3-yl, these compounds can be prepared as described in *PCT Int. Appl.* WO 2008005964.

If it is desired to produce the compound of formula X, where R^{3a} is 1-difluoromethyl-1*H-*pyrazol-3-yl, this compound can be prepared as described in *PCT Int. Appl.* WO 2005090332.

The carboxylic acid of the compounds of formula IX and the amines of formula X may be converted to the compounds of formula I through any conventional means to form an amide bond between a carboxylic acid and an amine (see for example, Montalbetti, C. A. G. N., Falque, V., Tetrahedron, 2005, 61, 10827-10852). If the compounds of formula I are a mixture of enantiomers or diastereomers, the appropriate chromatographic techniques, such as supercritical fluid chromatography, may be utilized to produce chirally pure or chirally enriched compounds of formula I. If R³ contains any protecting group functionality, that functionality may be removed via standard deprotection methods.

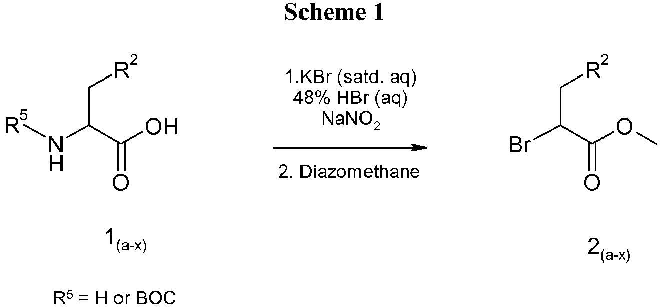

Compounds 2(a-x) can be synthesized following the reactions outlined in Scheme 1. The amino acid or protected amino acid, compound 1(a-x), can be converted to a diazonium species and then converted *in situ* to the bromide under standard conditions (see for example, Archer, C. H., Thomas, N. R., Gani, D. Tet. Asymm., 1993, 4(6), 1141-1152; Dener, J. M., Zhang, L.-H., Rapoport, H. J. Org. Chem., 1993, 58, 1159-1166; Souers, A. J., Schurer, S., Kwack, H., Virgilio, A. A., Ellman, J. A, Synthesis, 1999, 4, 583-585). The resulting halo-acid can either be maintained as the acid or can then be converted to an appropriately functionalized ester by any conventional method of converting an acid to an ester as described in reaction Scheme 1 (see for example, Archer, C. H., Thomas, N. R., Gani, D. Tet. Asymm., 1993, 4(6), 1141-1152).

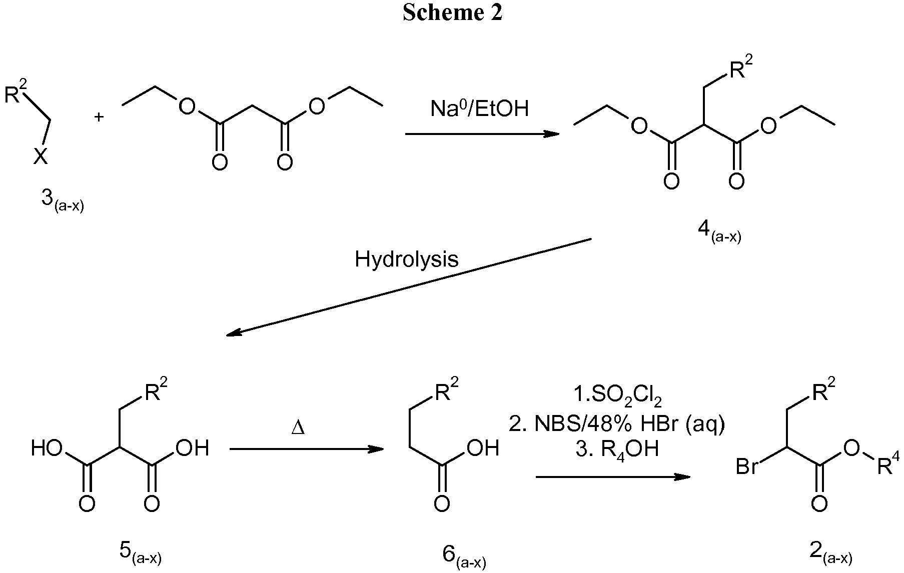

Compounds 2(a-x) can be synthesized following the reactions outlined in Scheme 2. The compounds of formula 3(a-x), where X is halogen or any functional group that may be displaced or coupled through a carbon, may be purchased or produced from commercially available material under standard conditions (see for example, Fujimoto, R.A., Francis, J.E., Hutchison, A.J. in U.S. patent, US4977144; Kortylewicz, Z.P., Galardy, R.E., J. Med. Chem., 1990, 33, 263-273). Compound 3(a-x) may then be reacted with a malonate derivative under standard conditions to produce a substituted malonate (see for example, Kortylewicz, Z.P., Galardy, R.E., J. Med. Chem., 1990, 33, 263-273). The resulting substituted malonate, compounds 4(a-x), can then be treated under hydrolysis conditions to form the resulting diacids (see for example, Kortylewicz, Z.P., Galardy, R.E., J. Med. Chem., 1990, 33, 263-273). The diacids of compounds 5(a-x) can then be heated under such conditions that will promote a decarboxylation to form the appropriately substituted acids. (see for example, Kortylewicz, Z.P., Galardy, R.E., J. Med. Chem., 1990, 33, 263-273). In some instances, the substituted acids of compounds 6(a-x) may be available from commercial sources. The resulting substituted acids, compounds 6(a-x), may then be treated under standard conditions to produce acid chlorides followed by *in situ* generation of the adjacent bromides (see for example, Epstein, J.W., Brabander, H.J., Fanshawe, W.J., Hofmann, C.M., McKenzie, T.C., Safir, S.R., Osterberg, A.C., Cosulich, D.B., Lovell, F.M., J. Med.Chem., 1981, 24, 481-490). The acid chlorides can then be treated with an appropriate alcohol, to form compounds 2(a-x) as described in reaction Scheme 2.

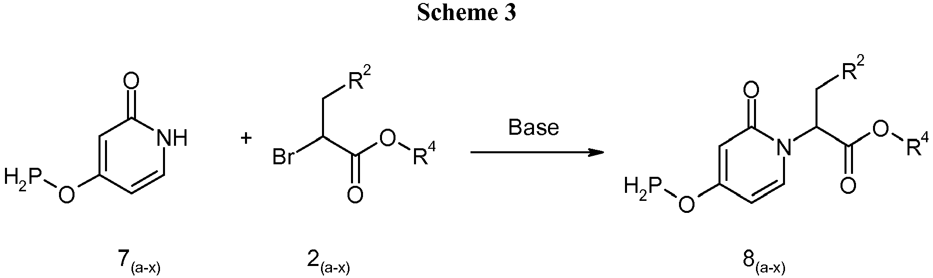

Compounds 8(a-x) can be synthesized following the reaction outlined in Scheme 3. The compound of formula 7(a-x), where P is a protecting group, preferably benzyl, is readily available from commercial sources or can be readily prepared from commercially available 2,4-dihydroxypyridine (see for example, *PCT Int. Appl.* WO2008/022979 A1). Compounds 7(a-x) can be treated under standard deprotonation conditions and then further reacted with compounds 2(a-x) to afford compounds 8(a-x) (see for example, *PCT Inter. Appl.* WO 2003/068230 A1).

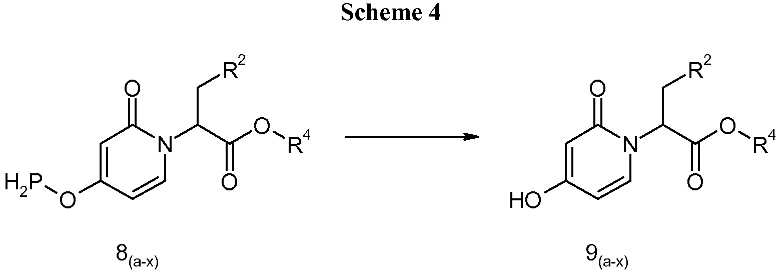

Compounds 9(a-x) can be synthesized following the reaction outlined in Scheme 4. The compounds of formula 9(a-x) may be produced from the compounds of formula 8(a-x) through standard methods to remove oxygen linked protecting groups (see for example, *PCT Inter. Appl.* WO 2003/068230 A1; Greene, T. W. Protective Groups in Organic Synthesis; John Wiley & Sons, Inc.: New York, 1991).

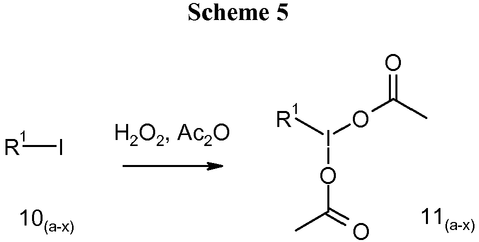

Compounds 11(a-x) can be synthesized following the reaction outlined in Scheme 5. The compound of formula 11(a-x) may be produced from the commercially available starting materials, 1 0(a-x), where R₁ is preferably an aromatic or heteroaromatic system, using standard conditions as described in reaction Scheme 5 (see for example, Molecules, 2005, 10, 190-194).

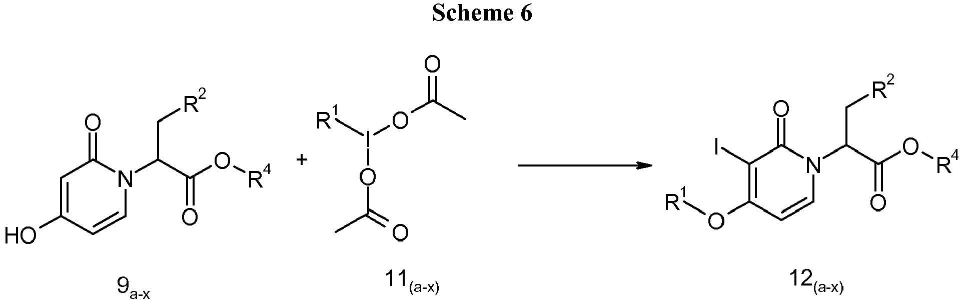

Compounds 12(a-x) can be synthesized following the reaction outlined in Scheme 6. The compounds of formula 12(a-x) may be produced from the compounds of formula 9(a-x) and the compounds of formula 11(a-x) using standard conditions (see for example, Bioorg. Med. Chem. Lett., 2007, 17, 712-716; Bioorg. Med. Chem. Lett., 2003, 13, 4309-4312).

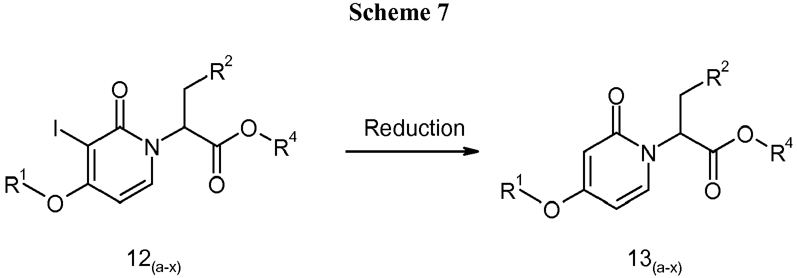

Compounds 13(a-x) can be synthesized following the reaction outlined in Scheme 7. The compounds of formula 13(a-x) may be produced from the compounds of formula 12(a-x) using standard reduction conditions (see for example, Anorganische Chemie, 1983, 38, 398 - 403).

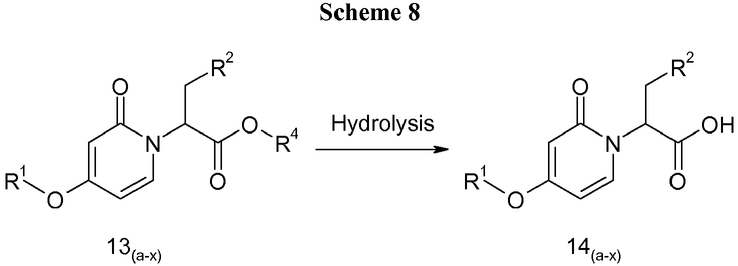

Compounds 14(a-x) can be synthesized following the reaction outlined in Scheme 8. The compounds of formula 14(a-x) may be produced from the compounds of formula 13(a-x) using conventional methods to convert an ester to a carboxylic acid, preferably via hydrolysis (see for example, New, J.S., Christopher, W.L., Jass, P.A., J. Org. Chem., 1989, 54, 990-992).

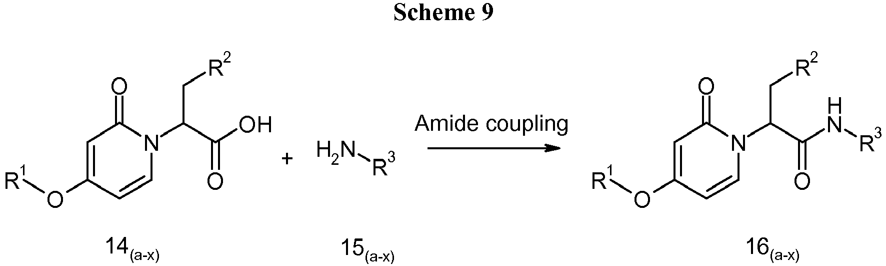

Compounds 16(a-x) may be synthesized following the reaction outlined in Scheme 9. The carboxylic acids, compounds 14(a-x), and the appropriate commercially available or synthetically accessible amines may be treated under standard amide bond formation conditions to afford compounds 16(a-x) (see for example, Montalbetti, C. A. G. N., Falque, V., Tetrahedron, 2005, 61, 10827-10852). Final deprotection or chemical conversion of 16(a-x) may be required to produce the desired fmal compound.

### EXAMPLES

### Example 1

### 2-[4-(2,3-Difluoro-phenoxy)-2-oxo-2H-pyridin-1-yl]-4-methyl-pentanoic acid (1-methyl-1H-pyrazol-3-yl)-amide

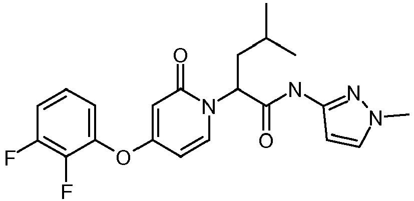

Preparation of 2-bromo-4-methyl-pentanoic acid methyl ester: A solution of 4-methyl-pentanoic acid (50 g, 0.43 mol) in carbon tetrachloride (200 mL) at 25°C was treated with thionyl chloride (125 mL, 1.72 mol). The reaction was then heated to 65°C and stirred for 30 min. After this time, the reaction was removed from the heat and was then treated with N-bromosuccinimide (100 g, 0.56 mol), carbon tetrachloride (200 mL) and a 48% aqueous hydrogen bromide (40 drops). The reaction was then heated to 85°C and stirred overnight. After this time, the reaction was cooled to 0°C and carefully quenched with methanol (150 mL) until no further gas evolution was observed. The mixture was then filtered and washed with hexanes. The dark solution was concentrated *in vacuo.* The remaining liquid was then partitioned between water (300 mL) and hexanes (3 × 300 mL). The combined organics were washed with a saturated aqueous sodium bicarbonate solution, dried over anhydrous sodium sulfate, filtered, rinsed and concentrated *in vacuo.* The obtained residue was distilled *in vacuo* to afford 2-bromo-4-methyl-pentanoic acid methyl ester as colorless oil (60 g, 66%); ¹H NMR (300 MHz, *CDCl₃*): δ 4.28 (t, 1H, *J=* 7.5 Hz), 3.77 *(s,* 3H), 1.90 (t, 2H, *J=* 7.5 Hz), 1.78 - 1.71 (*m*, 1H)□0.93 (*dd,* 6H, *J₁* = 14.1 Hz, *J₂* = 6.6 Hz). GC-MS: 209 [M]⁺, t_{R} = 5.50 min.

Preparation of 2-(4-benzyloxy-2-oxo-2H-pyridin-1-yl)-4-methyl-pentanoic acid methyl ester: A solution of 4-benzyloxy-1H-pyridin-2-one (15 g, 74.6 mmol) in *N,N,-*dimethylformamide (200 mL) was treated with 2-bromo-4-methyl-pentanoic acid methyl ester (29.2 g, 112 mmol, 80% purity) and potassium carbonate (15.5 g, 112 mmol). The mixture was stirred at 80°C overnight. At this time, the reaction was allowed to cool to 25°C and was then concentrated to dryness *in vacuo.* The residue was washed with ethyl acetate (300 mL) and the remaining solid was removed by filtration. The filtrate was concentrated *in vacuo.* Flash column chromatography (ethyl acetate:petroleum ether: 1:2) afforded 2-(4-benzyloxy-2-oxo-2H-pyridin-1-yl)-4-methyl-pentanoic acid methyl ester (18 g, 73%); ¹H NMR (300 MHz, *CDCl₃*): δ 7.41-7.36 (*m*, 5H), 7.21 (d, 1H, *J=* 7.8 Hz), 6.06 - 6.00 (*m*, 2H), 5.7 (*dd,* 1H, *J*₁ = 10.5 Hz, *J₂* = 5.4 Hz), 4.99(s, 2H), 3.71(*s*, 3H), 1.96 - 1.86 (*m*, 2H), 1.49 - 1.42 (*m*, 1H), 0.96 *(d,* 3H, J= 6.6 Hz), 0.93 (d, 3H, J= 6.9 Hz),. LC-MS: 330 [M + 1]⁺, t_{R} = 2.71 min.

Preparation of 2-(4-hydroxy-2-oxo-2H-pyridin-1-yl)-4-methyl-pentanoic acid methyl ester: A solution of 2-(4-benzyloxy-2-oxo-2H-pyridin-1-yl)-4-methyl-pentanoic acid methyl ester (6.4 g, 19.5 mmol) in methanol (100 mL) was treated with 10% palladium on carbon (0.6 g). The reaction mixture was stirred overnight under an atmosphere of H₂. At this time, the reaction mixture was filtered to remove the catalyst. The filtrate was concentrated *in vacuo* to afford 2-(4-hydroxy-2-oxo-2H-pyridin-1-yl)-4-methyl-pentanoic acid methyl ester (4.4 g, 94%) as white solid. ¹H NMR (300 MHz, *d₆*-DMSO): δ 10.69 *(s,* 1H), 7.51 *(d,* 1H, J= 7.8 Hz), 5.91 (*dd,* 1H, *J₁* = 7.5 Hz, *J₂* = 2.1 Hz), 5.57 *(d,* 1H, J= 2.4 Hz), 5.26 *(dd,* 1H, *J₁* = 11.4 Hz, *J₂* = 4.2 Hz), 3.62 *(s,* 3H), 2.06 - 1.77 (*m*, 2H), 1.30 - 1.26 (*m*, 1H), 0.86 - 0.83 (*m*, 6H). LC-MS: 240 [M + 1]⁺, t_{R} = 1.48 min.

Preparation of 1,2-difluoro-3-iodobenzene diacetate: A solution of hydrogen peroxide (4.2 mL) and acetic anhydride (18.3 mL) was stirred at 40°C for 4 h. At this time, the reaction was treated with 1,2-difluoro-3-iodo-benzene (5.0 g, 20.8 mmol). The resulting solution was stirred at 40°C overnight. At this time, the solvent was removed *in vacuo.* The residue was washed with ether to afford 1,2-difluoro-3-iodobenzene diacetate (1.9 g, 25.5 %); ¹H NMR (300 MHz, *CDCl₃*): δ 7.90 -7.86 (*m*, 1H), 7.44 - 7.41 (*m*, 1H), 7.32 - 7.27 *(s,* 1H), 2.00 *(s,* 6H).

Preparation of 2-[4-(2,3-difluoro-phenoxy)-3-iodo-2-oxo-2H-pyridin-1-yl]-4-methyl-pentanoic acid methyl ester: A solution of sodium carbonate (500 mg, 4.6 mmol) and 2-(4-hydroxy-2-oxo-2H-pyridin-1-yl)-4-methyl-pentanoic acid methyl ester (1.0 g, 4.2 mmol) in water (50 mL) at 25°C was treated with 1,2-difluoro-3-iodobenzene diacetate (1.5 g, 4.2 mmol). The mixture was stirred at 25°C for 30 min. At this time, the mixture was extracted with ethyl acetate (3 x 30 mL). The combined organic layers were concentrated *in vacuo.* The resulting residue was dissolved in *N,N*-dimethylformamide (30 mL) and then heated to reflux for 30 min. After cooling to 25°C, the solution was evaporated to dryness to afford 2-[4-(2,3-difluoro-phenoxy)-3-iodo-2-oxo-2H-pyridin-1-yl]-4-methyl-pentanoic acid methyl ester (2.0 g). This material was used witout further purification; LC-MS: 478 [M+1]⁺, t_{R} = 3.03 min

Preparation of 2-[4-(2,3-difluoro-phenoxy)-2-oxo-2H-pyridin-1-yl]-4-methyl-pentanoic acid methyl ester: A solution of 2-[4-(2,3-difluoro-phenoxy)-3-iodo-2-oxo-2H-pyridin-1-yl]-4-methyl-pentanoic acid methyl ester (2.0 g, crude) in acetic acid (30 mL) was treated with zinc (1 g, 15 mmol). The mixture was stirred at 30°C overnight. At this time, the solids were removed by filtration. The filtrated was concentrated *in vacuo.* The resulting residue was dissolved in ethyl acetate (40 mL) and was washed with water (1 × 20 mL). The organics were dried over anhydrous sodium sulfate, filtered and concentrated *in vacuo* to afford 2-[4-(2,3-difluoro-phenoxy)-2-oxo-2H-pyridin-1-yl]-4-methyl-pentanoic acid methyl ester (1.2 g). This material was used witout further purification; LC-MS: 352 [M+1]⁺, t_{R} = 1.64 min.

Preparation of 2-[4-(2,3-difluoro-phenoxy)-2-oxo-2H-pyridin-1-yl]-4-methyl-pentanoic acid: A solution of 2-[4-(2,3-difluoro-phenoxy)-2-oxo-2H-pyridin-1-yl]-4-methyl-pentanoic acid methyl ester (1.2 g, crude) in methanol (30 mL) was treated with a 4M solution of aqueous sodium hydroxide (2.1 mL). The reaction was stirred at 25°C overnight. At this time, the reaction was concentrated in *vacuo.* The residue was dissolved in water (10 mL) and then was extracted with ethyl acetate (1 × 10 mL). The aqueous layer was acidified with aqueous hydrochloric acid. The precipitate which resulted was collected by filtration and then was washed with ether to afford 2-[4-(2,3-difluoro-phenoxy)-2-oxo-2H-pyridin-1-yl]-4-methyl-pentanoic acid (900 mg, 63.6 % for three steps); ¹H NMR (300 MHz, *d₆*-DMSO): δ 13.14 (s, 1H, broad), 7.79 (d, 1H, J= 7.8 Hz), 7.51 - 7.29 (*m*, 3H), 6.26 - 6.22 (*m*, 1H), 5.57 *(s,* 1H), 5.32 (*dd,* 1H, *J₁* = 10.5 Hz, *J₂* = 4.8 Hz), 2.08 - 1.85 (*m,* 2H), 1.30 *(s,* 1H, broad), 0.88 *(d,* 6H, J= 6.6 Hz). LC-MS: 338 [M+1]⁺, t_{R} = 2.52 min. HPLC: 100.00 % at 214 nm, 99.43 % at 254 nm, t_{R} = 5.58 min.

Preparation of 2-[4-(2,3-difluoro-phenoxy)-2-oxo-2H-pyridin-1-yl]-4-methyl-pentanoic acid (1-methyl-1H-pyrazol-3-yl)-amide: A solution of 2-[4-(2,3-difluoro-phenoxy)-2-oxo-2H-pyridin-1-yl]-4-methyl-pentanoic acid (100 mg, 0.3 mmol) in *N,N*-dimethylformamide (2 mL) at 25°C was treated with *N,N*-diisopropyethylamine (0.15 mL, 0.9 mmol), O-(7-azabenzotriazol-1-yl)-*N,N,N'N'*-tetramethyluronium hexafluorophosphate (HATU) (171 mg, 0.45 mmol) and 1-methyl-1H-pyrazol-3-ylamine (32 mg, 0.33 mmol). The reaction was stirred at 25°C overnight. At this time, the reaction was diluted with ethyl acetate (30 mL) and was washed with a saturated aqueous ammonium chloride solution (20 mL), a saturated aqueous sodium bicarbonate solution (20 mL) and a saturated aqueous sodium chloride solution (20 mL), dried over anhydrous sodium sulfate, filtered, rinsed and concentrated *in vacuo.* Silica gel column chromatography (100% ethyl acetate) afforded 2-[4-(2,3-difluoro-phenoxy)-2-oxo-2H-pyridin-1-yl]-4-methyl-pentanoic acid (1-methyl-1H-pyrazol-3-yl)-amide (95 mg, 76%) as a white solid; ES⁺-HRMS m/e calcd for C₂₁H₂₂N₄O₃F₂ [M+H⁺] 417.1733, found 417.1733; ¹H NMR (400 MHz, DMSO-*d*₆) δ ppm 10.96 (s, 1 H), 7.83 (d, *J*=7.9 Hz, 1 H), 7.55 (d, *J*=2.0 Hz, 1 H), 7.40-7.49 (m, 1 H), 7.23 - 7.37 (m, 2 H), 6.38 (d, *J*=2.0 Hz, 1 H), 6.22 (dd, *J*=7.9, 2.8 Hz, 1 H), 5.74 (dd, *J*=11.6, 4.9 Hz, 1 H), 5.55 (d, *J*=2.8 Hz, 1 H), 3.74 (s, 3 H), 1.97 - 2.07 (m, 1 H), 1.77 (ddd, *J*=14.2, 9.6, 4.9 Hz, 1 H), 1.23 - 1.37 (m, 1 H), 0.88 (d, *J*=6.6 Hz, 3 H), 0.85 (d, *J*=6.6 Hz, 3 H).

### Example 2

### 2-[4-(3-Fluoro-phenoxy)-2-oxo-2H-pyridin-1-yl]-4-methyl-pentanoic acid (1-methyl-1H-pyrazol-3-yl)-amide

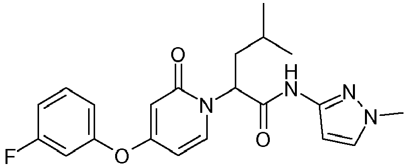

In an analogous manner to the stepwise sequence outlined in Example 1, starting from 1-fluoro-3-iodo-benzene afforded 2-[4-(3-fluoro-phenoxy)-2-oxo-2H-pyridin-1-yl]-4-methyl-pentanoic acid (1-methyl-1H-pyrazol-3-yl)-amide as a white solid (101 mg, 71% for the final step); ES⁺-HRMS m/e calcd for C₂₁H₂₃N₄O₃F [M+H⁺] 399.1827, found 399.1828. ¹H NMR (400 MHz, DMSO-*d*₆) δ ppm 10.95 (s, 1 H), 7.80 (d, *J*=7.7 Hz, 1 H), 7.55 (d, *J*=2.1 Hz, 1 H), 7.49-7.57 (m, 1 H), 7.14 - 7.24 (m, 2 H), 7.08 (dd, *J*=8.1, 1.5 Hz, 1 H), 6.65 - 7.05 (m, 1 H), 6.38 (d, *J*=2.1 Hz, 1 H), 6.14 (dd, *J*=7.7, 2.8 Hz, 1 H), 5.74 (dd, *J*=11.4, 4.8 Hz, 1 H), 5.51 (d, *J*=2.8 Hz, 1 H), 3.74 (s, 3 H), 1.92 - 2.07 (m, 1 H), 1.77 (ddd, *J*=14.1, 9.5, 4.8 Hz, 1 H), 1.24 - 1.39 (m, 1 H), 0.89 (d, *J*=6.6 Hz, 3 H), 0.85 (d, *J*=6.6 Hz, 3 H).

### Example 3

### 4-Methyl-2-(2-oxo-4-phenoxy-2H-pyridin-1-yl)-pentanoic acid (1-methyl-1H-pyrazol-3-yl)-amide

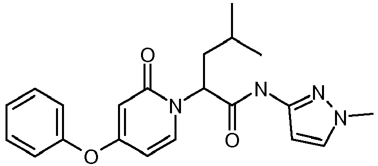

In an analogous manner to the stepwise sequence outlined in Example 1, starting from iodo-benzene afforded 4-methyl-2-(2-oxo-4-phenoxy-2H-pyridin-1-yl)-pentanoic acid (1-methyl-1H-pyrazol-3-yl)-amide as a white solid (66 mg, 58% for the final step); ES⁺-HRMS m/e calcd for C₂₁H₂₄N₄O₃ [M+H⁺] 381.1921, found 381.1921. ¹H NMR (400 MHz, DMSO-*d*₆) δ ppm 10.94 (s, 1 H), 7.78 (d, *J*=7.8 Hz, 1 H), 7.55 (d, *J*=1.9 Hz, 1 H), 7.50 (t, *J*=7.7 Hz, 2 H), 7.32 (t, *J*=7.7 Hz, 1 H), 7.21 (d, *J*=7.7 Hz, 2 H), 6.38 (d, *J*=1.9 Hz, 1 H), 6.13 (dd, *J*=7.8, 2.7 Hz, 1 H), 5.73 (dd, *J*=11.5, 4.9 Hz, 1 H), 5.41 (d, *J*=2.7 Hz, 1 H), 3.74 (s, 3 H), 1.90 - 2.08 (m, 1 H), 1.76 (ddd, *J*=14.0, 9.5, 4.9 Hz, 1 H), 1.31 (br. s., 1 H), 0.88 (d, *J*=6.6 Hz, 3 H), 0.85 (d, *J*=6.6 Hz, 3 H).

### Example 4

### 4-Methyl-2-(2-oxo-4-o-tolyloxy-2H-pyridin-1-yl)-pentanoic acid (1-methyl-1H-pyrazol-3-yl)-amide

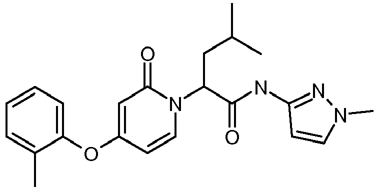

In an analogous manner to the stepwise sequence outlined in Example 1, starting from 1-iodo-2-methyl-benzene afforded 4-methyl-2-(2-oxo-4-o-tolyloxy-2H-pyridin-1-yl)-pentanoic acid (1-methyl-1H-pyrazol-3-yl)-amide as a white solid (121 mg, 77% for the final step); ES⁺-HRMS m/e calcd for C₂₂H₂₆N₄O₃ [M+H⁺] 395.2078, found 395.2078. ¹H NMR (400 MHz, DMSO-*d*₆) δ ppm 10.93 (s, 1 H), 7.78 (d, *J*=7.7 Hz, 1 H), 7.55 (d, *J*=2.2 Hz, 1 H), 7.38 (d, *J*=7.2 Hz, 1 H), 7.31 (td, *J*=7.9, 1.5 Hz, 1 H), 7.24 (td, *J*=7.2, 0.9 Hz, 1 H), 7.13 (d, *J*=7.9 Hz, 1 H), 6.38 (d, *J*=2.2 Hz, 1 H), 6.13 (dd, *J*=7.7, 2.8 Hz, 1 H), 5.72 (dd, *J*=11.3, 4.9 Hz, 1 H), 5.27 (d, *J*=2.8 Hz, 1 H), 3.73 (s, 3 H), 2.13 (s, 3 H), 1.92 - 2.04 (m, 1 H), 1.77 (ddd, *J*=14.1, 9.4, 4.9 Hz, 1 H), 1.24 - 1.38 (m, 1 H), 0.88 (d, *J*=6.6 Hz, 3 H), 0.85 (d, *J*=6.6 Hz, 3 H).

### Example 5

### 2-[4-(2,6-Difluoro-phenoxy)-2-oxo-2H-pyridin-1-yl]-4-methyl-pentanoic acid (1-methyl-1H-pyrazol-3-yl)-amide

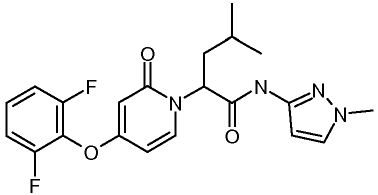

In an analogous manner to the stepwise sequence outlined in Example 1, starting from 1,3-difluoro-2-iodo-benzene afforded 2-[4-(2,6-difluoro-phenoxy)-2-oxo-2H-pyridin-1-yl]-4-methyl-pentanoic acid (1-methyl-1H-pyrazol-3-yl)-amide as a white solid (105 mg, 84% for the fmal step); ES⁺-HRMS m/e calcd for C₂₁H₂₂N₄O₃F₂ [M+H⁺] 417.1733, found 417.1732. ¹H NMR (400 MHz, DMSO-*d*₆) δ ppm 10.97 (s, 1 H), 7.84 (d, *J*=7.8 Hz, 1 H), 7.55 (d, *J*=2.1 Hz, 1 H), 7.41 - 7.50 (m, 1 H), 7.34 - 7.40 (m, 2 H), 6.38 (d, *J*=2.1 Hz, 1 H), 6.25 (dd, *J*=7.8, 2.9 Hz, 1 H), 5.73 (dd, *J*=11.5, 4.9 Hz, 1 H), 5.50 (d, *J*=2.8 Hz, 1 H), 3.74 (s, 3 H), 1.96 - 2.08 (m, 1 H), 1.78 (ddd, *J*=14.2, 9.6, 4.9 Hz, 1 H), 1.31 (m, 1 H), 0.88 (d, *J*=6.6 Hz, 3 H), 0.85 (d, *J*=6.6 Hz, 3 H).

### Example 6

### 4-Methyl-2-[2-oxo-4-(2-trifluoromethyl-phenoxy)-2H-pyridin-1-yl]-pentanoic acid (1-methyl-1H-pyrazol-3-yl)-amide

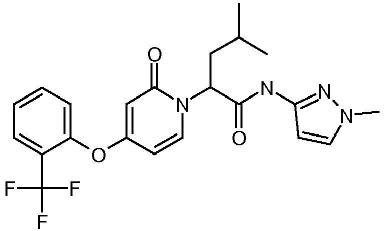

In an analogous manner to the stepwise sequence outlined in Example 1, starting from 1-iodo-2-trifluoromethyl-benzene afforded 4-methyl-2-[2-oxo-4-(2-trifluoromethyl-phenoxy)-2H-pyridin-1-yl]-pentanoic acid (1-methyl-1H-pyrazol-3-yl)-amide as a white solid (111 mg, 82% for the fmal step); ES⁺-HRMS m/e calcd for C₂₂H₂₃N₄O₃F₃ [M+Na⁺] 471.1614, found 471.1615. ¹H NMR (400 MHz, DMSO-*d*₆) δ ppm 10.97 (s, 1 H), 7.87 (d, *J*=7.9 Hz, 1 H), 7.82 (d, *J*=7.9 Hz, 1 H), 7.75 - 7.82 (m, 1 H), 7.79 (t, *J*=7.9 Hz, 1 H), 7.56 (d, *J*=2.0 Hz, 1 H), 7.52 (t, *J*=7.9 Hz, 1 H), 7.47 (d, *J*=7.9 Hz, 1 H), 6.38 (d, *J*=2.0 Hz, 1 H), 6.16 (dd, *J*=7.9, 2.7 Hz, 1 H), 5.74 (dd, *J*=11.3, 4.9 Hz, 1 H), 5.50 (d, *J*=2.7 Hz, 1 H), 3.74 (s, 3 H), 1.94 - 2.07 (m, 1 H), 1.77 (ddd, *J*=14.1, 9.5, 4.9 Hz, 1 H), 1.31 (br. s., 1 H), 0.89 (d, *J*=6.6 Hz, 3 H), 0.85 (d, *J*=6.6 Hz, 3 H).

### Example 7

### 2-[4-(4-Fluoro-phenoxy)-2-oxo-2H-pyridin-1-yl]-4-methyl-pentanoic acid (1-methyl-1H-pyrazol-3-yl)-amide

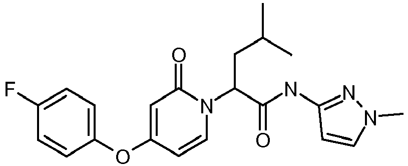

In an analogous manner to the stepwise sequence outlined in Example 1, starting from 1-fluoro-4-iodo-benzene afforded 2-[4-(4-fluoro-phenoxy)-2-oxo-2H-pyridin-1-yl]-4-methyl-pentanoic acid(1-methyl-1H-pyrazol-3-yl)-amide as a white solid (102 mg, 82% for the final step); ES⁺-HRMS m/e calcd for C₂₁H₂₃N₄O₃F [M+Na⁺] 421.1646, found 421.1643. ¹H NMR (400 MHz, DMSO-*d*₆) δ ppm 10.93 (s, 1 H), 7.78 (d, *J*=7.8 Hz, 1 H), 7.55 (d, *J*=2.0 Hz, 1 H), 7.24 - 7.37 (m, 4 H), 6.38 (d, *J*=2.0 Hz, 1 H), 6.13 (dd, *J*=7.8, 2.8 Hz, 1 H), 5.73 (dd, *J*=11.5, 4.9 Hz, 1 H), 5.40 (d, *J*=2.8 Hz, 1 H), 3.74 (s, 3 H), 1.94 - 2.06 (m, 1 H), 1.76 (ddd, *J*=14.1, 9.5, 4.9 Hz, 1 H), 1.23 - 1.37 (m, 1 H), 0.88 (d, *J*=6.6 Hz, 3 H), 0.84 (d, *J*=6.6 Hz, 3 H).

### Example 8

### 4-Methyl-2-[2-oxo-4-(pyridin-3-yloxy)-2H-pyridin-1-yl]-pentanoic acid (1-methyl-1H-pyrazol-3-yl)-amide

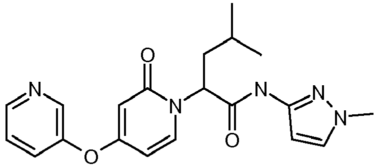

In an analogous manner to the stepwise sequence outlined in Example 1, starting from 3-iodo-pyridine afforded 4-methyl-2-[2-oxo-4-(pyridin-3-yloxy)-2H-pyridin-1-yl]-pentanoic acid (1-methyl-1H-pyrazol-3-yl)-amide as a white solid (143 mg, 94% for the final step); ES⁺-HRMS m/e calcd for C₂₀H₂₃N₅O₃ [M+H⁺] 382.1874, found 382.1874. ¹H NMR (400 MHz, DMSO-*d*₆) δ ppm 10.95 (s, 1 H), 8.52 - 8.55 (m, 2 H), 7.82 (d, *J*=7.9 Hz, 1 H), 7.75 (ddd, *J*=8.3, 2.6, 1.1 Hz, 1 H), 7.55 (d, *J*=2.2 Hz, 1 H), 7.52 - 7.57 (m, 1 H), 6.38 (d, *J*=2.2 Hz, 1 H), 6.17 (dd, *J*=7.9, 2.8 Hz, 1 H), 5.74 (dd, *J*=11.7, 4.9 Hz, 1 H), 5.47 (d, *J*=2.8 Hz, 1 H), 3.74 (s, 3 H), 1.94 - 2.06 (m, 1 H), 1.77 (ddd, *J*=14.2, 9.6, 4.9 Hz, 1 H), 1.25 - 1.38 (m, 1 H), 0.89 (d, *J*=6.6 Hz, 3 H), 0.85 (d, *J*=6.6 Hz, 3 H).

### Example 9

### 2-[4-(Biphenyl-2-yloxy)-2-oxo-2H-pyridin-1-yl]-4-methyl-pentanoic acid (1-methyl-1H-pyrazol-3-yl)-amide

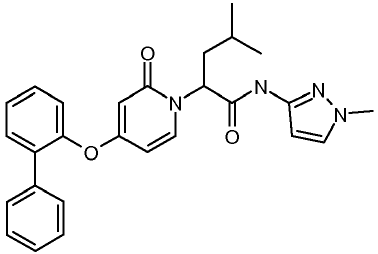

In an analogous manner to the stepwise sequence outlined in Example 1, starting from 2-iodo-biphenyl afforded 2-[4-(biphenyl-2-yloxy)-2-oxo-2H-pyridin-1-yl]-4-methyl-pentanoic acid (1-methyl-1H-pyrazol-3-yl)-amide as a white solid (106 mg, 77% for the fmal step); ES⁺-HRMS m/e calcd for C₂₇H₂₈N₄O₃ [M+H⁺] 457.2234, found 457.2235. ¹H NMR (400 MHz, DMSO-*d*₆) δ ppm 10.90 (s, 1 H), 7.67 (d, *J*=7.8 Hz, 1 H), 7.52 - 7.57 (m, 2 H), 7.49 (td, *J*=7.5, 1.9 Hz, 1 H), 7.42 - 7.47 (m, 3 H), 7.36 - 7.41 (m, 2 H), 7.33 (d, *J*=7.2 Hz, 1 H), 7.29 (d, *J*=8.1 Hz, 1 H), 6.35 (d, *J*=2.1 Hz, 1 H), 6.02 (dd, *J*=7.8, 2.8 Hz, 1 H), 5.66 (dd, *J*=11.5, 4.9 Hz, 1 H), 5.32 (d, *J*=2.8 Hz, 1 H), 3.73 (s, 3 H), 1.83 - 1.97 (m, 1 H), 1.71 (ddd, *J*=14.0, 9.3, 4.9 Hz, 1 H), 1.12 - 1.25 (m, 1 H), 0.84 (d, *J*=6.6 Hz, 2 H), 0.80 (d, *J*=6.6 Hz, 2 H)

### Example 10

### 2-[4-(2-Isopropyl-phenoxy)-2-oxo-2H-pyridin-1-yl]-4-methyl-pentanoic acid (1-methyl-1H-pyrazol-3-yl)-amide

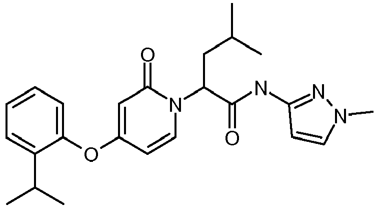

In an analogous manner to the stepwise sequence outlined in Example 1, starting from 1-iodo-2-isopropyl-benzene afforded 2-[4-(2-isopropyl-phenoxy)-2-oxo-2H-pyridin-1-yl]-4-methyl-pentanoic acid (1-methyl-1H-pyrazol-3-yl)-amide as a white solid (100 mg, 82% for the fmal step); ES⁺-HRMS m/e calcd for C₂₄H₃₀N₄O₃ [M+H⁺] 423.2391, found 423.2390. ¹H NMR (400 MHz, DMSO-*d*₆) δ ppm 10.95 (s, 1 H), 7.78 (d, *J*=7.8 Hz, 1 H), 7.55 (d, *J*=2.1 Hz, 1 H), 7.43 - 7.49 (m, 1 H), 7.26 - 7.33 (m, 2 H), 7.10 (m, 1 H), 6.38 (d, *J*=2.1 Hz, 1 H), 6.15 (dd, *J*=7.7, 2.8 Hz, 1 H), 5.72 (dd, *J*=11.3, 4.9 Hz, 1 H), 5.31 (d, *J*=2.8 Hz, 1 H), 3.74 (s, 3 H), 2.91 - 3.07 (m, 1 H), 1.99 (s, 1 H), 1.76 (ddd, *J*=14.1, 9.4, 4.9 Hz, 1 H), 1.32 (br. s., 1 H), 1.15 (d, *J*=6.8 Hz, 6 H), 0.88 (d, *J*=6.6 Hz, 3 H), 0.84 (d, *J*=6.6 Hz, 3 H).

### Example 11

### 2-[4-(2,4-Difluoro-phenoxy)-2-oxo-2H-pyridin-1-yl]-4-methyl-pentanoic acid (1-methyl-1H-pyrazol-3-yl)-amide

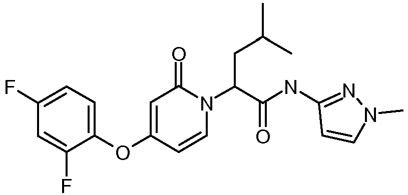

In an analogous manner to the stepwise sequence outlined in Example 1, starting from 2,4-difluoro-1-iodo-benzene afforded 2-[4-(2,4-difluoro-phenoxy)-2-oxo-2H-pyridin-1-yl]-4-methyl-pentanoic acid (1-methyl-1H-pyrazol-3-yl)-amide as a white solid (99 mg, 79% for the fmal step); ES⁺-HRMS m/e calcd for C₂₁H₂₂N₄O₃F₂ [M+H⁺] 417.1733, found 417.1732. ¹H NMR (400 MHz, DMSO-*d*₆) δ ppm 10.94 (s, 1 H), 7.80 (d, *J*=7.9 Hz, 1 H), 7.47 - 7.59 (m, 3 H), 7.21 (t, *J*=8.6 Hz, 1 H), 6.38 (d, *J*=2.1 Hz, 1 H), 6.18 (dd, *J*=7.9, 2.8 Hz, 1 H), 5.73 (dd, *J*=11.6, 4.8 Hz, 1 H), 5.44 (d, *J*=2.8 Hz, 1 H), 3.74 (s, 3 H), 1.95 - 2.07 (m, 1 H), 1.77 (ddd, *J*=14.2, 9.6, 4.8 Hz, 1 H), 1.20 - 1.37 (m, 1 H), 0.88 (d, *J*=6.6 Hz, 3 H), 0.85 (d, *J*=6.6 Hz, 3 H).

### Example 12

### 2-[4-(3-Chloro-2-methyl-phenoxy)-2-oxo-2H-pyridin-1-yl]-4-methyl-pentanoic acid (1-methyl-1H-pyrazol-3-yl)-amide

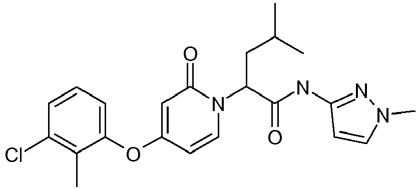

In an analogous manner to the stepwise sequence outlined in Example 1, starting from 1-chloro-3-iodo-2-methyl-benzene afforded 2-[4-(3-chloro-2-methyl-phenoxy)-2-oxo-2H-pyridin-1-yl]-4-methyl-pentanoic acid (1-methyl-1H-pyrazol-3-yl)-amide as a white solid (75 mg, 60% for the fmal step); ES⁺-HRMS m/e calcd for C₂₂H₂₅N₄O₃Cl [M+H⁺] 429.1688, found 429.1687. ¹H NMR (400 MHz, DMSO-*d*₆) δ ppm 10.95 (s, 1 H), 7.81 (d, *J*=7.9 Hz, 1 H), 7.55 (d, *J*=2.1 Hz, 1 H), 7.44 (d, *J*=7.9 Hz, 1 H), 7.34 (t, J=7.9 Hz, 1 H), 7.18 (d, *J*=7.9 Hz, 1 H), 6.38 (d, *J*=2.1 Hz, 1 H), 6.16 (dd, *J*=7.9, 2.8 Hz, 1 H), 5.73 (dd, *J*=11.4, 5.0 Hz, 1 H), 5.33 (d, *J*=2.8 Hz, 1 H), 3.74 (s, 3 H), 2.17 (s, 3 H), 1.94 - 2.05 (m, 1 H), 1.77 (ddd, *J*=14.1, 9.5, 5.0 Hz, 1 H), 1.23 - 1.39 (m, 1 H), 0.88 (d, *J*=6.6 Hz, 3 H), 0.85 (d, *J*=6.6 Hz, 3 H).

### Example 13

### 2-[4-(2,5-Difluoro-phenoxy)-2-oxo-2H-pyridin-1-yl]-4-methyl-pentanoic acid (1-methyl-1H-pyrazol-3-yl)-amide

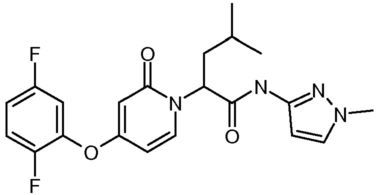

In an analogous manner to the stepwise sequence outlined in Example 1, starting from 1,4-difluoro-2-iodo-benzene afforded 2-[4-(2,5-difluoro-phenoxy)-2-oxo-2H-pyridin-1-yl]-4-methyl-pentanoic acid (1-methyl-1H-pyrazol-3-yl)-amide as a white solid (94 mg, 75% for the fmal step); ES⁺-HRMS m/e calcd for C₂₁H₂₂N₄O₃F₂ [M+H⁺] 417.1733, found 417.1734. ¹H NMR (400 MHz, DMSO-*d*₆) δ ppm 10.95 (s, 1 H), 7.82 (d, *J*=7.8 Hz, 1 H), 7.44 - 7.57 (m, 3 H), 7.22 - 7.30 (m, 1 H), 6.38 (d, *J*=2.1 Hz, 1 H), 6.20 (dd, *J*=7.8, 2.8 Hz, 1 H), 5.74 (dd, *J*=11.5, 4.9 Hz, 1 H), 5.52 (d, *J*=2.8 Hz, 1 H), 3.74 (s, 3 H), 1.95 - 2.07 (m, 1 H), 1.77 (ddd, *J*=14.2, 9.5, 4.9 Hz, 1 H), 1.23 - 1.39 (m, 1 H), 0.88 (d, *J*=6.6 Hz, 3 H), 0.85 (d, *J*=6.6 Hz, 3 H).

### Example 14

### 4-Methyl-2-[4-(naphthalen-1-yloxy)-2-oxo-2H-pyridin-1-yl]-pentanoic acid (1-methyl-1Hpyrazol-3-yl)-amide

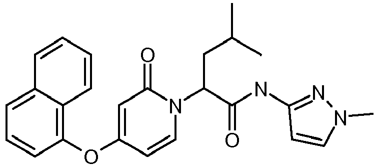

In an analogous manner to the stepwise sequence outlined in Example 1, starting from 1-iodo-naphthalene afforded 4-methyl-2-[4-(naphthalen-1-yloxy)-2-oxo-2H-pyridin-1-yl]-pentanoic acid (1-methyl-1H-pyrazol-3-yl)-amide as a white solid (106 mg, 82% for the final step); ES⁺-HRMS m/e calcd for C₂₅H₂₆N₄O₃ [M+H⁺] 431.2078, found 431.2078. ¹H NMR (400 MHz, DMSO-*d*₆) δ ppm 10.93 (s, 1 H), 8.06 (d, *J*=7.2 Hz, 1 H), 7.93 (d, *J*=8.3 Hz, 1 H), 7.85 (d, *J*=7.7 Hz, 1 H), 7.82 (d, *J*=7.9 Hz, 1 H), 7.56 - 7.65 (m, 3 H), 7.55 (d, *J*=2.0 Hz, 1 H), 7.40 (d, *J*=7.2 Hz, 1 H), 6.38 (d, *J*=2.0 Hz, 1 H), 6.24 (dd, *J*=7.7, 2.8 Hz, 1 H), 5.72 (dd, *J*=11.4, 5.0 Hz, 1 H), 5.34 (d, *J*=2.8 Hz, 1 H), 3.73 (s, 3 H), 1.93 - 2.06 (m, 1 H), 1.71 - 1.81 (m, 1 H), 1.33 (br. s., 1 H), 0.89 (d, *J*=6.6 Hz, 3 H), 0.84 (d, *J*=6.6 Hz, 3 H).

### Example 15

### 2-[4-(2-Chloro-phenoxy)-2-oxo-2H-pyridin-1-yl]-4-methyl-pentanoic acid (1-methyl-1H-pyrazol-3-yl)-amide

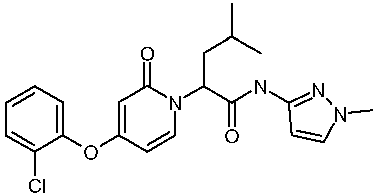

In an analogous manner to the stepwise sequence outlined in Example 1, starting from 1-chloro-2-iodo-benzene afforded 2-[4-(2-chloro-phenoxy)-2-oxo-2H-pyridin-1-yl]-4-methyl-pentanoic acid (1-methyl-1H-pyrazol-3-yl)-amide as a white solid (85 mg, 68% for the final step); ES⁺-HRMS m/e calcd for C₂₁H₂₃N₄O₃Cl [M+H⁺] 415.1532, found 415.1531. ¹H NMR (400 MHz, DMSO-*d*₆) δ ppm 10.96 (s, 1 H), 7.81 (d, *J*=7.8 Hz, 1 H), 7.67 (dd, J=7.9, 1.3 Hz, 1 H), 7.55 (d, *J*=2.0 Hz, 1 H), 7.45 - 7.51 (m, 1 H), 7.34 - 7.44 (m, 2 H), 6.38 (d, *J*=2.0 Hz, 1 H), 6.16 (dd, *J*=7.8, 2.8 Hz, 1 H), 5.73 (dd, *J*=11.5, 4.9 Hz, 1 H), 5.32 (d, *J*=2.8 Hz, 1 H), 3.74 (s, 3 H), 1.93 - 2.05 (m, 1 H), 1.77 (ddd, *J*=14.0, 9.5, 4.9 Hz, 1 H), 1.32 (br. s., 1 H), 0.88 (d, *J*=6.6 Hz, 3 H), 0.85 (d, *J*=6.6 Hz, 3 H).

### Example 16

### 4-Methyl-2-[2-oxo-4-(2-trifluoromethoxy-phenoxy)-2H-pyridin-1-yl]-pentanoic acid (1-methyl-1H-pyrazol-3-yl)-amide

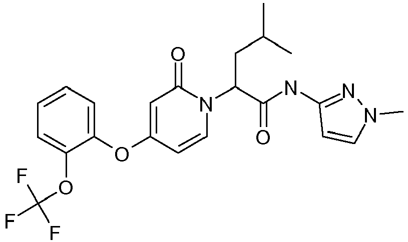

In an analogous manner to the stepwise sequence outlined in Example 1, starting from 1-iodo-2-trifluoromethoxy-benzene afforded 4-methyl-2-[2-oxo-4-(2-trifluoromethoxy-phenoxy)-2H-pyridin-1-yl]-pentanoic acid (1-methyl-1H-pyrazol-3-yl)-amide as a white solid (89 mg, 64% for the fmal step); ES⁺-HRMS m/e calcd for C₂₂H₂₃N₄O₄F₃ [M+Na⁺] 487.1563, found 457.1564. ¹H NMR (400 MHz, DMSO-*d*₆) δ ppm 10.96 (s, 1 H), 7.82 (d, *J*=7.9 Hz, 1 H), 7.61 (d, *J*=8.1 Hz, 1 H), 7.55 (d, *J*=2.1 Hz, 1 H), 7.42 - 7.53 (m, 3 H), 6.38 (d, *J*=2.1 Hz, 1 H), 6.16 (dd, *J*=7.9, 2.8 Hz, 1 H), 5.74 (dd, *J*=11.5, 4.9 Hz, 1 H), 5.44 (d, *J*=2.8 Hz, 1 H), 3.74 (s, 3 H), 1.94 - 2.06 (m, 1 H), 1.77 (ddd, *J*=14.2, 9.5, 4.9 Hz, 1 H), 1.22 - 1.38 (m, 1 H), 0.88 (d, *J*=6.6 Hz, 3 H), 0.85 (d, *J*=6.6 Hz, 3 H).

### Example 17

### 2-[4-(2-Fluoro-phenoxy)-2-oxo-2H-pyridin-1-yl]-4-methyl-pentanoic acid (1-methyl-1H-pyrazol-3-yl)-amide

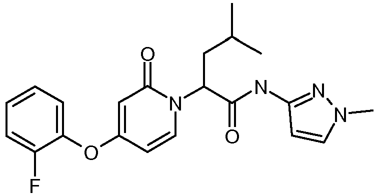

In an analogous manner to the stepwise sequence outlined in Example 1, starting from 1-fluoro-2-iodo-benzene afforded 2-[4-(2-fluoro-phenoxy)-2-oxo-2H-pyridin-1-yl]-4-methyl-pentanoic acid (1-methyl-1H-pyrazol-3-yl)-amide as a white solid (84 mg, 68% for the final step); ES⁺-HRMS m/e calcd for C₂₁H₂₃N₄O₃F [M+H⁺] 399.1827, found 399.1826. ¹H NMR (400 MHz, DMSO-*d*₆) δ ppm 10.95 (s, 1 H), 7.81 (d, *J*=7.9 Hz, 1 H), 7.55 (d, *J*=2.0 Hz, 1 H), 7.29-7.50 (m, 4 H), 6.38 (d, *J*=2.0 Hz, 1 H), 6.18 (dd, *J*=7.9, 2.8 Hz, 1 H), 5.73 (dd, *J*=11.6, 4.8 Hz, 1 H), 5.41 (d, *J*=2.8 Hz, 1 H), 3.74 (s, 3 H), 1.95 - 2.06 (m, 1 H), 1.77 (ddd, *J*=14.2, 9.6, 4.8 Hz, 1 H), 1.23 - 1.39 (m, 1 H), 0.88 (d, *J*=6.6 Hz, 3 H), 0.85 (d, *J*=6.6 Hz, 3 H).

### Example 18

### 2-[4-(2-Methoxy-phenoxy)-2-oxo-2H-pyridin-1-yl]-4-methyl-pentanoic acid (1-methyl-1H-pyrazol-3-yl)-amide

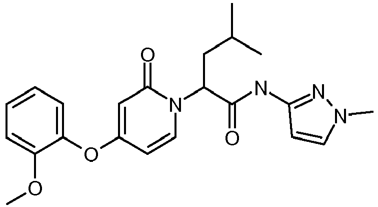

In an analogous manner to the stepwise sequence outlined in Example 1, starting from 1-iodo-2-methoxy-benzene afforded 2-[4-(2-methoxy-phenoxy)-2-oxo-2H-pyridin-1-yl]-4-methyl-pentanoic acid (1-methyl-1H-pyrazol-3-yl)-amide as a white solid (103 mg, 83% for the final step); ES⁺-HRMS m/e calcd for C₂₂H₂₆N₄O₄ [M+H⁺] 411.2027, found 411.2026. ¹H NMR (400 MHz, DMSO-*d*₆) δ ppm 10.92 (s, 1 H), 7.74 (d, *J*=7.9 Hz, 1 H), 7.55 (d, *J*=2.0 Hz, 1 H), 7.27-7.36 (m, 1 H), 7.22 (dd, *J*=8.2, 1.4 Hz, 1 H), 7.19 (dd, *J*=7.8, 1.4 Hz, 1 H), 7.02 (td, *J*=7.8, 1.4 Hz, 1 H), 6.37 (d, *J*=2.0 Hz, 1 H), 6.11 (dd, *J*=7.9, 2.8 Hz, 1 H), 5.71 (dd, *J*=11.4, 5.0 Hz, 1 H), 5.27 (d, *J*=2.8 Hz, 1 H), 3.77 (s, 3 H), 3.73 (s, 3 H), 1.93 - 2.04 (m, 1 H), 1.76 (ddd, *J*=14.2, 9.4, 5.0 Hz, 1 H), 1.31 (br. s., 1 H), 0.88 (d, *J*=6.6 Hz, 3 H), 0.84 (d, *J*=6.6 Hz, 3 H).

### Example 19

### In Vitro Glucokinase Activity

The compounds of formula I which include the compounds set forth in the Examples activated glucokinase *in vitro* by the procedure of this Example. In this manner, they increase the flux of glucose metabolism which causes increased insulin secretion. Therefore, the compounds of formula I are glucokinase activators useful for increasing insulin secretion.

Glucokinase In Vitro Assay Protocol: Glucokinase (GK) was assayed by coupling the production of glucose-6-phosphate to the generation of NADH with glucose-6-phosphate dehydrogenase (G6PDH, 0.75-1 kunits/mg; Boehringer Mannheim, Indianapolis, IN) from *Leuconostoc mesenteroides* as the coupling enzyme:

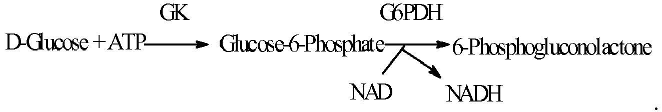

Recombinant human liver GK1 was expressed in *E*. *coli* as a glutathione S-transferase fusion protein (GST-GK) [Liang et al, 1995] and was purified by chromatography over a glutathione-Sepharose 4B affinity column using the procedure provided by the manufacturer (Amersham Pharmacia Biotech, Piscataway, NJ). Previous studies have demonstrated that the enzymatic properties of native GK and GST-GK are essentially identical (Liang et al, 1995; Neet et al., 1990).

The assay was conducted at 30°C in a flat bottom 96-well tissue culture plate from Costar (Cambridge, MA) with a fmal incubation volume of 120 µL. The incubation reaction contained the following: 25 mM Hepes buffer (pH 7.1), 25 mM KCl, 5 mM D-glucose, 1mM ATP, 1.8 mM NAD, 2 mM MgCl₂, 1 µM sorbitol-6-phosphate, 1 mM dithiothreitol, test drug or 10% DMSO, ∼7 units/ml G6PDH, and GK (see below). All organic reagents were >98% pure and were from Boehringer Mannheim with the exceptions of D-glucose and Hepes which were from Sigma Chemical Co, St Louis, MO. Test compounds were dissolved in DMSO and were added to the incubation reaction minus GST-GK in a volume of 12 µL to yield a fmal DMSO concentration of 10%. This mix was pre-incubated in the temperature controlled chamber of a SPECTRAmax 250 microplate spectrophotometer (Molecular Devices Corporation, Sunnyvale, CA) for 10 minutes to allow temperature equilibrium and then the reaction was started by the addition of 20 µL GST-GK.

After addition of enzyme, the increase in optical density (OD) at 340 nm was monitored spectrophotometrically to determine the rate of change (OD₃₄₀ per min). The GK activity (OD₃₄₀/min) in control wells (10% DMSO minus GK activators) was compared with the activity in wells containing test GK activators, and the concentration of activator that produced a 50% increase in the activity of GK, i.e., the SC_{1.5}, was calculated. The table below provides the *in vitro* glucokinase activity for the compounds in the Examples:

| **Example** | **SC1.5 average (µM)** |
|---|---|
| 1 | 10.4 |
| 2 | >30 |
| 3 | 21.7 |
| 4 | 7.3 |
| 5 | 4.2 |
| 6 | 11.4 |
| 7 | >30 |
| 8 | 26.1 |
| 9 | >30 |
| 10 | 4.5 |
| 11 | 22.5 |
| 12 | 9.0 |
| 13 | 16.6 |
| 14 | 2.6 |
| 15 | 6.2 |
| 16 | 8.8 |
| 17 | 8.4 |
| 18 | 8.1 |

### References:

Liang, Y., Kesavan, P., Wang, L., Niswender, K., Tanizawa, Y., Permut, M. A., Magnuson, M., and Matschinsky, F. M. Variable effects of maturity-onset-diabetes-of- youth (MODY)-associated glucokinase mutations on the substrate interactions and stability of the enzyme. Biochem. J. 309: 167-173, 1995.
Neet, K., Keenan, R. P., and Tippett, P.S. Observation of a kinetic slow transition in monomeric glucokinase. Biochemistry 29; 770-777, 1990.

## Claims

1. A compound of formula (I) 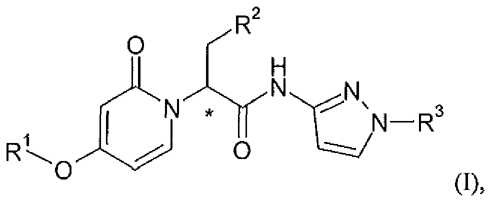 wherein
R¹ is aryl, said aryl being unsubstituted or mono-, bi- or tri-substituted independently with halogen, phenyl, C₁-C₆alkyl, C₁-C₆halogenalkyl, C₁-C₆alkoxy or C₁-C₆halogenalkoxy, or
heteroaryl, said heteroaryl being unsubstituted or substituted with halogen or C₁-C₆alkyl, or
R² is selected from the group consisting of C₁-C₆alkyl, C₁-C₆halogenalkyl, and cycloalkyl, said cycloalkyl being unsubstituted or substituted by halogen, oxo or hydroxy; and
R³ is C₁-C₆alkyl or C₁-C₆halogenalkyl;
or pharmaceutically acceptable salts thereof.

2. The compound according to claim 1, wherein R¹ is phenyl, unsubstituted or mono-, bi- or tri-substituted independently with mono-, bi- or tri-substituted independently with halogen, phenyl, C₁-C₆alkyl C₁-C₆halogenalkyl, C₁-C₆alkoxy or C₁-C₆halogenalkoxy.

3. The compound according to claims 1 orrr 2, wherein R¹ is phenyl, unsubstituted or substituted with fluorine, chlorine, phenyl, -CF₃, -CH₃, -OCF₃, -OCH₃ or -CH(CH₃)₂.

4. The compound according to any one of claims 1 to 3, wherein R¹ is phenyl bi-substituted with fluorine or phenyl substituted with both chlorine and -CH₃,

5. The compound according to claims 1 or 4, wherein R¹ is pyridine, unsubstituted or substituted with halogen or C₁-C₆alkyl.

6. The compound according to claim 1, wherein R¹ is naphthalen-1-yl.

7. The compound according to any one of claims 1 to 6, wherein R² is C₁-C₆alkyl.

8. The compound according to any one of claims 1 to 7, wherein R² is -CH(CH₃)₂.

9. The compound according to any one of claims 1 to 8, wherein R³ is C₁-C₆alkyl.

10. The compound according to any one of claims 1 to 9, wherein R³ is methyl.

11. The compound according to claim 1, wherein said compound is selected from the group consisting of
2-[4-(2,3-difluoro-phenoxy)-2-oxo-2H-pyridin-1-yl]-4-methyl-pentanoic acid (1-methyl-1H-pyrazol-3-yl)-amide;
2-[4-(3-fluoro-phenoxy)-2-oxo-2H-pyridin-1-yl]-4-methyl-pentanoic acid (1-methyl-1H-pyrazol-3-yl)-amide;
4-methyl-2-(2-oxo-4-phenoxy-2H-pyridin-1-yl)-pentanoic acid (1-methyl-1H-pyrazol-3-yl)-amide;
4-methyl-2-(2-oxo-4-o-tolyloxy-2H-pyridin-1-yl)-pentanoic acid (1-methyl-1H-pyrazol-3-yl)-amide;
2-[4-(2,6-difluoro-phenoxy)-2-oxo-2H-pyridin-1-yl]-4-methyl-pentanoic acid (1-methyl-1H-pyrazol-3-yl)-amide;
4-methyl-2-[2-oxo-4-(2-trifluoromethyl-phenoxy)-2H-pyridin-1-yl]-pentanoic acid (1-methyl-1H-pyrazol-3-yl)-amide;
2-[4-(4-fluoro-phenoxy)-2-oxo-2H-pyridin-1-yl]-4-methyl-pentanoic acid (1-methyl-1H-pyrazol-3-yl)-amide;
4-methyl-2-[2-oxo-4-(pyridin-3-yloxy)-2H-pyridin-1-yl]-pentanoic acid (1-methyl-1H-pyrazol-3-yl)-amide;
2-[4-(biphenyl-2-yloxy)-2-oxo-2H-pyridin-1-yl]-4-methyl-pentanoic acid (1-methyl-1H-pyrazol-3-yl)-amide;
2-[4-(2-isopropyl-phenoxy)-2-oxo-2H-pyridin-1-yl]-4-methyl-pentanoic acid (1-methyl-1H-pyrazol-3-yl)-amide;
2-[4-(2,4-difluoro-phenoxy)-2-oxo-2H-pyridin-1-yl]-4-methyl-pentanoic acid (1-methyl-1H-pyrazol-3-yl)-amide;
2-[4-(3-chloro-2-methyl-phenoxy)-2-oxo-2H-pyridin-1-yl]-4-methyl-pentanoic acid (1-methyl-1H-pyrazol-3-yl)-amide;
2-[4-(2,5-difluoro-phenoxy)-2-oxo-2H-pyridin-1-yl]-4-methyl-pentanoic acid (1-methyl-1H-pyrazol-3-yl)-amide;
4-methyl-2-[4-(naphthalen-1-yloxy)-2-oxo-2H-pyridin-1-yl]-pentanoic acid (1-methyl-1H-pyrazol-3-yl)-amide;
2-[4-(2-chloro-phenoxy)-2-oxo-2H-pyridin-1-yl]-4-methyl-pentanoic acid (1-methyl-1H-pyrazol-3-yl)-amide;
4-methyl-2-[2-oxo-4-(2-trifluoromethoxy-phenoxy)-2H-pyridin-1-yl]-pentanoic acid (1-methyl-1H-pyrazol-3-yl)-amide;
2-[4-(2-fluoro-phenoxy)-2-oxo-2H-pyridin-1-yl]-4-methyl-pentanoic acid (1-methyl-1H-pyrazol-3-yl)-amide;
2-[4-(2-methoxy-phenoxy)-2-oxo-2H-pyridin-1-yl]-4-methyl-pentanoic acid (1-methyl-1H-pyrazol-3-yl)-amide,
or a pharmaceutically acceptable salt thereof.

12. A pharmaceutical composition, comprising a therapeutically effective amount of a compound of formula (I) according to any one of claims 1 to 11 and a pharmaceutically acceptable carrier and/or adjuvant.

13. The compound of formula (I) according to any one of claims 1 to 11 for use as therapeutic active substance.

14. The compound of formula (I) according to any one of claims 1 to 11 for use in the treatment of a metabolic disease and/or disorder.

15. The compound of formula (I) according to claim 14 for use in the treatment of diabetes mellitus.

## Patentansprüche

1. Eine Verbindung der Formel (I) 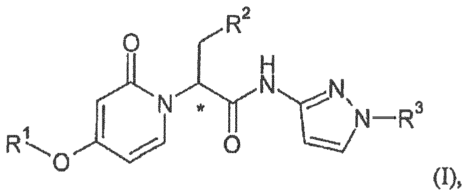 wobei
R¹ Aryl, wobei das Aryl unsubstituiert oder ein-, zwei- oder dreifach unabhängig mit Halogen, Phenyl, C₁-C₆-Alkyl, C₁-C₆-Halogenalkyl, C₁-C₆-Alkoxy oder C₁-C₆-Halogenalkoxy substituiert ist, oder
Heteroaryl, wobei das Heteroaryl unsubstituiert oder mit Halogen oder C₁-C₆-Alkyl substituiert ist, ist oder
R² aus der Gruppe bestehend aus C₁-C₆-Alkyl, C₁-C₆-Halogenalkyl und Cycloalkyl ausgewählt ist, wobei das Cycloalkyl unsubstituiert oder mit Halogen, Oxo oder Hydroxy substituiert ist; und
R³ C₁-C₆-Alkyl oder C₁-C₆-Halogenalkyl ist;
oder pharmazeutisch verträgliche Salze davon.

2. Die Verbindung nach Anspruch 1, wobei R¹ Phenyl, unsubstituiert oder ein-, zwei- oder dreifach unabhängig substituiert mit, ein-, zwei- oder dreifach unabhängig substituiert mit Halogen, Phenyl, C₁-C₆-Alkyl, C₁-C₆-Halogenalkyl, C₁-C₆-Alkoxy oder C₁-C₆-Halogenalkoxy, ist.

3. Die Verbindung nach den Ansprüchen 1 oder 2, wobei R¹ Phenyl, unsubstituiert oder substituiert mit Fluor, Chlor, Phenyl, -CF₃, -CH₃, -OCF₃, -OCH₃ oder -CH(CH₃)₂, ist.

4. Die Verbindung nach einem der Ansprüche 1 bis 3, wobei R¹ Phenyl, zweifach substituiert mit Fluor, oder Phenyl, substituiert mit sowohl Chlor als auch -CH₃, ist.

5. Die Verbindung nach den Ansprüchen 1 oder 4, wobei R¹ Pyridin, unsubstituiert oder substituiert mit Halogen oder C₁-C₆-Alkyl, ist.

6. Die Verbindung nach Anspruch 1, wobei R¹ Naphthalen-1-yl ist.

7. Die Verbindung nach einem der Ansprüche 1 bis 6, wobei R² gleich C₁-C₆-Alkyl ist.

8. Die Verbindung nach einem der Ansprüche 1 bis 7, wobei R² gleich -CH(CH₃)₂ ist.

9. Die Verbindung nach einem der Ansprüche 1 bis 8, wobei R³ gleich C₁-C₆-Alkyl ist.

10. Die Verbindung nach einem der Ansprüche 1 bis 9, wobei R³ Methyl ist.

11. Die Verbindung nach Anspruch 1, wobei die Verbindung ausgewählt ist aus der Gruppe bestehend aus
2-[4-(2,3-Difluor-phenoxy)-2-oxo-2H-pyridin-1-yl]-4-methyl-pentansäure-(1-methyl-1H-pyrazol-3-yl)-amid;
2-[4-(3-Fluor-phenoxy)-2-oxo-2H-pyridin-1-yl]-4-methyl-pentansäure-(1-methyl-1H-pyrazol-3-yl)-amid;
4-Methyl-2-(2-oxo-4-phenoxy-2H-pyridin-1-yl)-pentansäure-(1-methyl-1H-pyrazol-3-yl)-amid;
4-Methyl-2-(2-oxo-4-o-tolyloxy-2H-pyridin-1-yl)-pentansäure-(1-methyl-1H-pyrazol-3-yl)-amid;
2-[4-(2,6-Difluor-phenoxy)-2-oxo-2H-pyridin-1-yl]-4-methyl-pentansäure-(1-methyl-1H-pyrazol-3-yl)-amid;
4-Methyl-2-[2-oxo-4-(2-trifluormethyl-phenoxy)-2H-pyridin-1-yl]-pentansäure-(1-methyl-1 H-pyrazol-3-yl)-amid;
2-[4-(4-Fluor-phenoxy)-2-oxo-2H-pyridin-1-yl]-4-methyl-pentansäure-(1-methyl-1H-pyrazol-3-yl)-amid;
4-Methyl-2-[2-oxo-4-(pyridin-3-yloxy)-2H-pyridin-1-yl]-pentansäure-(1-methyl-1H-pyrazol-3-yl)-amid;
2-[4-(Biphenyl-2-yloxy)-2-oxo-2H-pyridin-1-yl]-4-methyl-pentansäure-(1-methyl-1H-pyrazol-3-yl)-amid;
2-[4-(2-Isopropyl-phenoxy)-2-oxo-2H-pyridin-1-yl]-4-methyl-pentansäure-(1-methyl-1H-pyrazol-3-yl)-amid;
2-[4-(2,4-Difluor-phenoxy)-2-oxo-2H-pyridin-1-yl]-4-methyl-pentansäure-(1-methyl-1H-pyrazol-3-yl)-amid;
2-[4-(3-Chlor-2-methyl-phenoxy)-2-oxo-2H-pyridin-1-yl]-4-methyl-pentansäure-(1-methyl-1H-pyrazol-3-yl)-amid;
2-[4-(2,5-Difluor-phenoxy)-2-oxo-2H-pyridin-1-yl]-4-methyl-pentansäure-(1-methyl-1H-pyrazol-3-yl)-amid;
4-Methyl-2-[4-(naphthalen-1-yloxy)-2-oxo-2H-pyridin-1-yl]-pentansäure-(1-methyl-1H-pyrazol-3-yl)-amid;
2-[4-(2-Chlor-phenoxy)-2-oxo-2H-pyridin-1-yl]-4-methyl-pentansäure-(1-methyl-1H-pyrazol-3-yl)-amid;
4-Methyl-2-[2-oxo-4-(2-trifluormethoxy-phenoxy)-2H-pyridin-1-yl]-pentansäure-(1-methyl-1H-pyrazol-3-yl)-amid;
2-[4-(2-Fluor-phenoxy)-2-oxo-2H-pyridin-1-yl]-4-methyl-pentansäure-(1-methyl-1H-pyrazol-3-yl)-amid;
2-[4-(2-Methoxy-phenoxy)-2-oxo-2H-pyridin-1-yl]-4-methyl-pentansäure-(1-methyl-1H-pyrazol-3-yl)-amid
oder ein pharmazeutisch verträgliches Salz davon.

12. Ein Arzneimittel, umfassend eine therapeutisch wirksame Menge einer Verbindung der Formel (I) nach einem der Ansprüche 1 bis 11 und einen pharmazeutisch verträglichen Träger und/oder Hilfsstoff.

13. Die Verbindung der Formel (I) nach einem der Ansprüche 1 bis 11 zur Verwendung als therapeutischer Wirkstoff.

14. Die Verbindung der Formel (I) nach einem der Ansprüche 1 bis 11 zur Verwendung bei der Behandlung einer Stoffwechselerkrankung und/oder -störung.

15. Die Verbindung der Formel (I) nach Anspruch 14 zur Verwendung bei der Behandlung von Diabetes mellitus.

## Revendications

1. Composé de formule (I) : 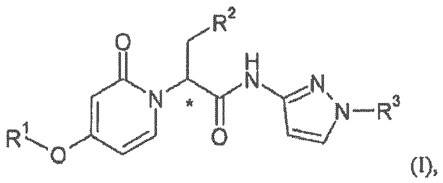 dans laquelle,
R¹ représente un groupe aryle, ledit groupe aryle étant non substitué ou mono-, bi- ou trisubstitué indépendamment avec un substituant halogéno, phényle, alkyle en C₁ à C₆, halogénoalkyle en C₁ à C₆, alkoxy en C₁ à C₆ ou halogénoalkoxy en C₁ à C₆,
ou
un groupe hétéroaryle, ledit groupe hétéroaryle étant non substitué ou substitué avec un substituant halogéno ou alkyle en C₁ à C₆, ou
R² est choisi dans le groupe consistant en des groupes alkyle en C₁ à C₆, halogénoalkyle en C₁ à C₆ et cycloalkyle, ledit groupe cycloalkyle étant non substitué ou substitué avec un substituant halogéno, oxo ou hydroxy ; et
R³ représente un groupe alkyle en C₁ à C₆ ou halogénoalkyle en C₁ à C₆ ; ou ses sels pharmaceutiquement acceptables.

2. Composé suivant la revendication 1, dans lequel R¹ représente un groupe phényle, non substitué ou mono-, *bi*- ou tri-substitué indépendamment avec mono-, bi- ou tri-substitué indépendamment avec un substituant halogéno, phényle, alkyle en C₁ à C₆, halogénoalkyle en C₁ à C₆, alkoxy en C₁ à C₆ ou halogénoalkoxy en C₁ à C₆.

3. Composé suivant la revendication 1 ou 2, dans lequel R¹ représente un groupe phényle, non substitué ou substitué avec un substituant fluoro, chloro, phényle, -CF₃, -CH₃, -OCF₃, -OCH₃ ou -CH(CH₃)₂.

4. Composé suivant l'une quelconque des revendications 1 à 3, dans lequel R¹ représente un groupe phényle bi-substitué avec un substituant fluoro ou phényle substitué avec à la fois un substituant chloro et un substituant -CH₃.

5. Composé suivant la revendication 1 ou 4, dans lequel R¹ représente un groupe pyridine, non substitué ou substitué avec un substituant halogéno ou alkyle en C₁ à C₆.

6. Composé suivant la revendication 1, dans lequel R¹ représente un groupe naphtalène-1-yle.

7. Composé suivant l'une quelconque des revendications 1 à 6, dans lequel R² représente un groupe alkyle en C₁ à C₆.

8. Composé suivant l'une quelconque des revendications 1 à 7, dans lequel R² représente un groupe -CH(CH₃)₂.

9. Composé suivant l'une quelconque des revendications 1 à 8, dans lequel R³ représente un groupe alkyle en C₁ à C₆.

10. Composé suivant l'une quelconque des revendications 1 à 9, dans lequel R³ représente un groupe méthyle.

11. Composé suivant la revendication 1, ledit composé étant choisi dans le groupe consistant en
le (1-méthyl-1H-pyrazole-3-yl)-amide d'acide 2-[4-(2,3-difluoro-phénoxy)-2-oxo-2H-pyridine-1-yl]-4-méthyl-pentanoïque;
le (1-méthyl-1H-pyrazole-3-yl)-amide d'acide 2-[4-(3-fluoro-phénoxy)-2-oxo-2H-pyridine-1-yl]-4-méthyl-pentanoïque ;
le (1-méthyl-1H-pyrazole-3-yl)-amide d'acide 4-méthyl-2-(2-oxo-4-phénoxy-2H-pyridine-1-yl)-pentanoïque ;
le (1-méthyl-1H-pyrazole-3-yl)-amide d'acide 4-méthyl-2-(2-oxo-4-o-tolyloxy-2H-pyridine-1-yl)-pentanoïque ;
le (1-méthyl-1H-pyrazole-3-yl)-amide d'acide 2-[4-(2,6-difluoro-phénoxy)-2-oxo-2H-pyridine-1-yl]-4-méthyl-pentanoïque ;
le (1-méthyl-1H-pyrazole-3-yl)-amide d'acide 4-méthyl-2-[2-oxo-4-(2-trifluorométhyl-phénoxy)-2H-pyridine-1-yl]-pentanoïque ;
le (1-méthyl-1H-pyrazole-3-yl)-amide d'acide 2-[4-(4-fluoro-phénoxy)-2-oxo-2H-pyridine-1-yl]-4-méthyl-pentanoïque ;
le (1-méthyl-1H-pyrazole-3-yl)-amide d'acide 4-méthyl-2-[2-oxo-4-(pyridine-3-yloxy)-2H-pyridine-1-yl]-pentanoïque;
le (1-méthyl-1H-pyrazole-3-yl)-amide d'acide 2-[4-(biphényl-2-yloxy)-2-oxo-2H-pyridine-1-yl]-4-méthyl-pentanoïque ;
le (1-méthyl-1H-pyrazole-3-yl)-amide d'acide 2-[4-(2-isopropyl-phénoxy)-2-oxo-2H-pyridine-1-yl]-4-méthyl-pentanoïque ;
le (1-méthyl-1H-pyrazole-3-1)-amide d'acide 2-[4-(2,4-difluoro-phénoxy)-2-oxo-2H-pyridine-1-yl]-4-méthyl-pentanoïque ;
le (1-méthyl-1H-pyrazole-3-yl)-amide d'acide 2-[4-(3-chloro-2-méthyl-phénoxy)-2-oxo-2H-pyridine-1-yl]-4-méthyl-pentanoïque ;
le (1-méthyl-1H-pyrazole-3-yl)-amide d'acide 2-[4-(2,5-difluoro-phénoxy)-2-oxo-2H-pyridine-1-yl]-4-méthyl-pentanoïque ;
le (1-méthyl-1H-pyrazole-3-yl)-amide d'acide 4-méthyl-2-[4-(naphtalène-1-yloxy)-2-oxo-2H-pyridine-1-yl]-pentanoïque ;
le (1-méthyl-1H-pyrazole-3-yl)-amide d'acide 2-[4-(2-chloro-phénoxy)-2-oxo-2H-pyridine-1-yl]-4-méthyl-pentanoïque ;
le (1-méthyl-1H-pyrazole-3-yl)-amide d'acide 4-méthyl-2-[2-oxo-4-(2-trifluorométhoxy-phénoxy)-2H-pyridine-1-yl]-pentanoïque ;
le (1-méthyl-1H-pyrazole-3-yl)-amide d'acide 2-[4-(2-fluoro-phénoxy)-2-oxo-2H-pyridine-1-yl]-4-méthyl-pentanoïque ;
le (1-méthyl-1H-pyrazole-3-yl)-amide d'acide 2-[4-(2-méthoxy-phénoxy)-2-oxo-2H-pyridine-1-yl]-4-méthyl-pentanoïque ;
ou un de ses sels pharmaceutiquement acceptables.

12. Composition pharmaceutique comprenant une quantité thérapeutiquement efficace d'un composé de formule (I) suivant l'une quelconque des revendications 1 à 11 et un support et/ou adjuvant pharmaceutiquement acceptable.

13. Composé de formule (I) suivant l'une quelconque des revendications 1 à 11 pour une utilisation comme substance thérapeutique active.

14. Composé de formule (I) suivant l'une quelconque des revendications 1 à 11 pour une utilisation dans le traitement d'une maladie et/ou d'un trouble métabolique.

15. Composé de formule (I) suivant la revendication 14, pour une utilisation dans le traitement du diabète sucré.
